(19) **Europäisches Patentamt / European Patent Office / Office européen des brevets**

(11) **EP 3 277 723 B1**

(12) # FASCICULE DE BREVET EUROPEEN

(45) Date de publication et mention de la délivrance du brevet:
**17.06.2020 Bulletin 2020/25**

(21) Numéro de dépôt: **16714843.6**

(22) Date de dépôt: **31.03.2016**

(51) Int Cl.:
**C07K 16/28** *(2006.01)*    **C12N 15/79** *(2006.01)*

(86) Numéro de dépôt international:
**PCT/EP2016/056994**

(87) Numéro de publication internationale:
**WO 2016/156449 (06.10.2016 Gazette 2016/40)**

(54) **LIGNEE CELLULAIRE SUREXPRIMANT L'ANTIGENE CD303 HUMAIN**

MENSCHLICHES CD303-ANTIGEN ÜBEREXPRIMIERENDE ZELLINIE

CELL LINE OVEREXPRESSING HUMAN CD303 ANTIGEN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **31.03.2015 FR 1552757**
**16.12.2015 FR 1562452**

(43) Date de publication de la demande:
**07.02.2018 Bulletin 2018/06**

(73) Titulaire: **Laboratoire Français du Fractionnement et des Biotechnologies 91940 Les Ulis (FR)**

(72) Inventeurs:
• **FOURNIER, Nathalie 59193 Erquinghem-Lys (FR)**
• **DE ROMEUF, Christophe 59130 Lambersart (FR)**

(74) Mandataire: **Regimbeau 20, rue de Chazelles 75847 Paris Cedex 17 (FR)**

(56) Documents cités:
• **CAO WEI ET AL: "BDCA2/Fc epsilon RI gamma complex signals through a novel BCR-like pathway in human plasmacytoid dendritic cells", PLOS BIOLOGY, vol. 5, no. 10, 1 octobre 2007 (2007-10-01), pages 2190-2200, XP055105679, DOI: DOI:10.1371/JOURNAL.PBIO.0050248**
• **J. RÖCK ET AL: "CD303 (BDCA-2) signals in plasmacytoid dendritic cells via a BCR-like signalosome involving Syk, Slp65 and PLCc2", EUROPEAN JOURNAL OF IMMUNOLOGY, vol. 37, no. 12, 1 décembre 2007 (2007-12-01), pages 3564-3575, XP055225344, DOI: 10.1002/eji.200737711**
• **DZIONEK A ET AL: "BDCA-2, a novel plasmacytoid dendritic cell-specific type II C-type lectin, mediates antigen capture and is a potent inhibitor of interferon alpha/beta induction", THE JOURNAL OF EXPERIMENTAL MEDICINE, ROCKEFELLER UNIVERSITY PRESS, US, vol. 194, no. 12, 17 décembre 2001 (2001-12-17), pages 1823-1834, XP002277387, ISSN: 0022-1007, DOI: 10.1084/JEM.194.12.1823**

Remarques:
Le dossier contient des informations techniques présentées postérieurement au dépôt de la demande et ne figurant pas dans le présent fascicule.

**Description**

**DOMAINE DE L'INVENTION**

La présente invention se situe dans le domaine des outils cellulaires destinés à caractériser l'activité *in vitro* ou *in vivo* des anticorps dirigés contre l'antigène CD303 humain, susceptibles d'être utilisés dans le traitement ou la prévention des tumeurs hématopoïétiques dérivées de cellules dendritiques plasmacytoïdes (pDC) ou des maladies inflammatoires, ou autoimmunes, impliquant les pDC. L'invention concerne en particulier des lignées cellulaires exprimant la chaine gamma du récepteur FcεRI et l'antigène CD303 humain, la lignée étant transfectée de façon stable par un vecteur d'expression comprenant une molécule d'acide nucléique codant pour l'antigène CD303 humain et présentant une forte expression du CD303 humain à sa surface, telles que revendiquées dans les revendications 1 à 4.

**ART ANTERIEUR**

Les cellules dendritiques (appelées « DC » dans toute la présente description) sont des cellules du système immunitaire présentatrices d'antigènes (CPA). Sous certaines conditions, les DC présentent des prolongements cytoplasmiques similaires aux dendrites des neurones.
Les cellules dendritiques ont deux fonctions principales :

- le déclenchement de la réponse immunitaire adaptative dirigée contre des antigènes du "non-soi" (antigènes étrangers), par présentation de ces antigènes étrangers aux lymphocytes T ; et
- le maintien de la tolérance centrale au "soi", par éducation des lymphocytes T dans le thymus, par le processus dit de « sélection négative ».

Les DC sont capables de se différencier en diverses sous-populations, selon les stimuli qu'elles reçoivent. Il existe trois grands types de cellules DC: les cellules DC conventionnelles, les cellules DC plasmacytoïdes (appelées « pDC ») et les cellules DC inflammatoires.
Les DC conventionnelles présentent les antigènes du soi ou du non-soi dans les organes lymphoïdes ou en périphérie. Les DC inflammatoires, probablement dérivées des monocytes sanguins, n'apparaissent qu'en cas de stimulation suite à une inflammation ou une infection.
Les DC plasmacytoïdes (pDC) sont circulantes, rondes et sans dendrites à l'état basal, mais acquièrent des dendrites après activation, généralement par un antigène viral. Elles produisent après stimulation une grande quantité d'interférons (IFN) de classe I, et sont principalement impliquées dans la réponse antivirale ou dans les maladies autoimmunes. Sur le plan phénotypique, elles sont notamment caractérisées par les marqueurs suivants : CD4+, CD11c-, Lin-, CD303+, CD304+.
Les pDC peuvent être à l'origine de tumeurs hématopoïétiques dans lesquelles elles acquièrent un marqueur supplémentaire (CD56). On parle alors de néoplasme de cellules dendritiques plasmacytoïdes blastiques (abrégé en « BPDCN » pour « Blastic plasmacytoid dendritic cells neoplasm »). On parle également de tumeurs hématopoïétiques CD4+CD56+. Ces tumeurs hématopoïétiques sont rares (1% des leucémies aiguës) et se présentent sous la forme de nodules cutanés associés à une lympho-adénopathie ou un gonflement de la rate et à une cytopénie fréquente. Les manifestations cutanées sont très rapidement suivies par une infiltration de la moelle osseuse. Il est désormais admis que les cellules hématopoïétiques à l'origine de ces tumeurs sont les pDC.
Il a été proposé dans WO2012/080642 d'utiliser des anticorps anti-BDCA-2, c'est-à-dire anti-CD303, pour le traitement de ces néoplasmes par déplétion des cellules tumorales.
Les pDC sont également impliquées dans certaines maladies inflammatoires, et notamment dans certaines maladies autoimmunes, en particulier par le biais de leur sécrétion d'IFN de type I.
Cependant, afin de traiter ces maladies de manière efficace, il est nécessaire de disposer d'anticorps monoclonaux adaptés et permettant chez l'homme une élimination la plus efficace possible des pDC. Pour cela, des anticorps chimériques ou humanisés avec une forte affinité pour l'antigène CD303 et des capacités effectrices (ADCC, CDC, phagocytose, signalisation via cross-link de CD303 par les Récepteurs Fc, en particulier le récepteur FcγRIIIa (également appelé CD16a) et/ou apoptose) leur permettant d'éliminer les pDC en conditions physiologiques sont nécessaires.
Actuellement, du fait d'un accès limité aux cellules de patients et afin de garantir une reproductibilité des tests, la caractérisation de l'efficacité des anticorps anti-CD303 *in vitro* et *in vivo* s'effectue généralement sur des lignées cellulaires telles que la lignée cellulaire CAL-1 ou équivalente, dérivées de cellules d'un patient humain souffrant de BPDCN.
Toutefois, les inventeurs se sont aperçus que les lignées disponibles n'expriment qu'un faible nombre de molécules CD303 présentes à leur surface, plus faible que celui de la majorité des cellules tumorales de patients souffrant de BPDCN, et que ces lignées cellulaires ne présentent de ce fait qu'un faible pouvoir discriminant vis-à-vis des anticorps anti-CD303. En effet, à trop faible densité antigénique, l'activité fonctionnelle des anticorps est trop faible pour pouvoir mettre en évidence des différences d'efficacité entre différents anticorps.

WO2014/093396A1 décrit la transfection de cellules CHO (cellules d'ovaire de hamster) par un vecteur d'expression comprenant une première molécule d'acide nucléique codant pour l'antigène CD303 humain et une deuxième molécule d'acide nucléique codant pour la chaine gamma du récepteur FcεRI humain, et l'utilisation de cette lignée dans un test ADCC avec des anticorps anti-CD303. Les résultats présentés suggèrent que la transfection de CD303 dans les cellules CHO n'est pas stable dans la durée (Exemple 30), ce qui est confirmé par l'article associé ultérieur Pellerin et al (EMBO Mol Med (2015)7:464-476, see *Supplementary Materials and Methods, Cloning of Human and Cynomolgus BDCA2 and Expression in CHO cells*).

Il existe donc un réel besoin pour la mise à disposition de nouvelles lignées cellulaires exprimant stablement l'antigène CD303 humain à leur surface, et notamment de lignées cellulaires ayant un pouvoir discriminant amélioré afin de pouvoir sélectionner les anticorps anti-CD303 présentant l'activité la plus importante.

## RESUME DE L'INVENTION

Selon un premier objet, la présente invention concerne une lignée cellulaire exprimant la chaîne gamma du récepteur FcεRI et l'antigène CD303 humain, caractérisée en ce que ladite lignée cellulaire est transfectée de façon stable par un vecteur d'expression comprenant une molécule d'acide nucléique codant pour l'antigène CD303 humain et présente une forte expression de CD303 à sa surface, caractérisée en ce que ladite lignée cellulaire présente au moins 10000 molécules du CD303 humain par cellule et en ce que ladite lignée cellulaire est :

a) une lignée cellulaire de cellules dendritiques plasmacytoïdes humaines (pDC) transfectée de façon stable par un vecteur d'expression comprenant une molécule d'acide nucléique codant pour l'antigène CD303 humain ; ou

b) une lignée de lymphocyte humain, transfectée de façon stable par un vecteur d'expression comprenant une première molécule d'acide nucléique codant pour l'antigène CD303 humain et une deuxième molécule d'acide nucléique codant pour la chaine gamma du récepteur FcεRI humain, ou par deux vecteurs d'expression comprenant chacun une molécule d'acide nucléique codant respectivement pour l'antigène CD303 humain et pour la chaine gamma du récepteur FcεRI humain.

La présente description divulgue également un vecteur d'expression comprenant une première molécule d'acide nucléique codant pour l'antigène CD303 humain et une deuxième molécule d'acide nucléique codant pour la chaine gamma du récepteur FcεRI humain.

La présente description divulgue également un kit comprenant deux vecteurs d'expression comprenant chacun une molécule d'acide nucléique codant respectivement pour l'antigène CD303 humain et pour la chaine gamma du récepteur FcεRI humain.

Selon un autre objet, l'invention concerne l'utilisation de la lignée cellulaire selon l'invention pour caractériser une activité fonctionnelle d'un anticorps dirigé contre l'antigène CD303 humain, préférentiellement une activité de type ADCC, sécrétion de cytokines, CDC, apoptose, et phagocytose ou pour comparer l'affinité de liaison de plusieurs anticorps anti-CD303 à l'antigène CD303 humain ou les épitopes de l'antigène CD303 humain reconnus par plusieurs anticorps anti-CD303.

Selon un autre mode de réalisation, l'invention concerne un procédé de test de l'activité ADCC d'un anticorps dirigé contre l'antigène CD303 humain, comprenant les étapes suivantes :

a) mise en contact de la lignée cellulaire selon l'invention avec un anticorps dirigé contre l'antigène CD303 humain et des cellules effectrices ;

b) incubation pendant une durée appropriée pour permettre la lyse des cellules de la lignée cellulaire selon l'invention de l'étape a); et

c) mesure du taux de lyse des cellules de la lignée cellulaire selon l'invention de l'étape a).

Selon un autre objet, l'invention consiste en un kit de caractérisation d'anticorps anti-CD303 comprenant la lignée cellulaire selon l'invention, et avantageusement au moins un réactif choisi parmi des cellules effectrices, des IgG polyvalentes, et des anticorps anti-CD303 de référence.

## DESCRIPTION DES FIGURES

**Figure 1.** Carte du vecteur pcDNA3.1 (-)

**Figure 2.** Expression de CD303 sur les cellules de l'ensemble 008-1 à jour 43. Les histogrammes montrent les cellules marquées avec un anticorps anti-CD303 conjugué PE (zone remplie) et avec un anticorps isotype contrôle conjugué PE (ligne).

**Figure 3.** Expression de CD303 sur les cellules de l'ensemble 008-1 après le 3ème tri cellulaire. Les histogrammes montrent les cellules colorées avec un anticorps anti-CD303 conjugué PE (zone remplie) et avec un anticorps isotype contrôle conjugué PE (ligne pointillée).

**Figure 4.** Expression de CD303 sur les cellules de l'ensemble 008-1 après le 3ème tri cellulaire et 2 mois de culture. Les histogrammes montrent les cellules colorées avec un anticorps anti-CD303 conjugué PE (zone remplie) et avec un anticorps isotype contrôle conjugué PE (ligne).

**Figure 5.** Expression de CD303 par des clones obtenus après clonage par dilution limite. Les histogrammes montrent les cellules marquées avec un anticorps anti-CD303 conjugué PE (à droite) et avec un anticorps isotype contrôle conjugué PE (à gauche).

**Figure 6.** Marquage du pool de cellules CAL-1 transfectées par un vecteur d'expression de CD303 avec un anticorps isotype contrôle PE ou un anticorps anti-CD303-PE à jour 22 après transfection.

**Figure 7.** Marquage des clones NF-3C8 et NF-5G9 obtenus après clonage à jour 50 après transfection avec un anticorps isotype contrôle PE ou un anticorps anti-CD303-PE.

**Figure 8.** Stabilité de la forte expression de CD303 par les clones NF-3C8 et NF-5G9 obtenus par transfection des cellules CAL-1 par un vecteur d'expression de CD303. Evolution du marquage par un anticorps anti-CD303 PE des clones NF-3C8 et NF-5G9 entre 2 et 12 semaines après repiquage en présence ou en absence de G418.

**Figure 9.** Stabilité de la forte expression de CD303 par les clones NF-3C8 et NF-5G9 obtenus par transfection des cellules CAL-1 par un vecteur d'expression de CD303. Marquage par un anticorps anti-CD303 PE des clones NF-3C8 (A) et NF-5G9 (B) à 2 et 12 semaines après repiquage en présence ou en absence de G418.

**Figure 10.** Liaison à l'antigène CD303 humain exprimé sur NF-3C8 ou NF-5G9 par les anticorps

**Figure 11.** Activité ADCC via CD16a induite par les anticorps chimériques anti-CD303 122A2, 114D1, ou 102E9, par un anticorps chimérique contrôle ou par l'anticorps humanisé anti-CD303 BIIB059 en présence de la lignée CAL-1 non transfectée par un vecteur d'expression de l'antigène CD303 humain (A) ou du clone NF-3C8 obtenu après transfection de la lignée CAL-1 par un vecteur d'expression de l'antigène CD303 humain, sélection et clonage (B).

## DESCRIPTION DETAILLEE DE L'INVENTION

***Lignée cellulaire exprimant la chaîne gamma du récepteur FcεRI et l'antigène CD303 humain, transfectée de façon stable par un vecteur d'expression comprenant une molécule d'acide nucléique codant pour l'antigène CD303 humain et présentant une forte expression de CD303 à sa surface***

La présente invention concerne une lignée cellulaire telle que définie dans les revendications 1 à 4. La présente description divulgue également une lignée cellulaire exprimant la chaîne gamma du récepteur FcεRI et l'antigène CD303 humain, caractérisée en ce que ladite lignée cellulaire est transfectée de façon stable par un vecteur d'expression comprenant une molécule d'acide nucléique codant pour l'antigène CD303 humain et présente à sa surface une forte expression de CD303.

L'antigène CD303 humain est le membre C de la 4ème famille de domaine lectine de type C et est également appelé CLEC4 (pour « C-type lectin domain family 4, member C ») ; DLEC; HECL; BDCA2; CLECSF7; CLECSF11; ou PRO34150 (voir le site EntrezGene pour le gène CLEC4). Il s'agit d'une glycoprotéine transmembranaire de type II de 213 acides aminés, comprenant un court domaine cytoplasmique sans motif de signalisation évident (acides aminés 1-21), une région transmembranaire (acides aminés 22-41), un domaine cou (acides aminés 42-82), et un domaine extracellulaire de reconnaissance de carbohydrates (CRD pour « carbohydrate récognition domain » ; acides aminés 83-213) (Dzionek et al-2001). La séquence de l'ARNm codant pour cette protéine peut être trouvée sur la base de données Genbank dans sa version du 14 février 2002 sous le n° d'accession AF293615.1 (SEQ ID NO :1), tandis que la séquence d'acides aminés est accessible sur la base de données Genbank dans sa version du 14 février 2002 sous le n° d'accession AAL37036.1 (SEQ ID NO :2).

Les inventeurs ont constaté que pour obtenir une lignée cellulaire exprimant de façon stable l'antigène CD303 humain à sa surface, l'expression par la lignée cellulaire de la chaine gamma du récepteur FcεRI humain est indispensable. Ainsi, pour une lignée cellulaire n'exprimant pas à l'origine cette chaine gamma du récepteur FcεRI humain, la co-transfection de la lignée cellulaire par un vecteur d'expression comprenant une molécule d'acide nucléique codant pour l'antigène CD303 humain et un autre vecteur d'expression comprenant une molécule d'acide nucléique codant pour la chaine gamma du récepteur FcεRI humain ou la transfection par un vecteur comprenant deux molécules d'acide nucléique codant l'une pour la chaine gamma du récepteur FcεRI humain et l'autre pour l'antigène CD303 humain est nécessaire pour obtenir une lignée cellulaire exprimant de façon stable l'antigène CD303 humain à sa surface. En revanche si la lignée d'origine exprime déjà la chaine gamma du récepteur FcεRI humain, la transfection d'un unique vecteur d'expression comprenant une molécule d'acide nucléique codant pour l'antigène CD303 humain est suffisante.

La séquence codant pour la chaine gamma du récepteur FcεRI humain est connue de l'homme du métier. Notamment, la séquence de l'ARNm codant pour cette protéine peut être trouvée sur la base de données Genbank dans sa version du 3 mai 2014 sous le n° d'accession NM_004106.1 (SEQ ID NO :3), tandis que la séquence d'acides aminés est accessible sur la base de données Genbank dans sa version du 3 mai 2014 sous le n° d'accession NP_004097.1 (SEQ ID NO : 4). Sur cette base, l'homme du métier sait concevoir des séquences nucléiques codant pour la chaine gamma du récepteur FcεRI humain susceptibles d'être insérées dans un vecteur d'expression approprié pour la transfection d'une lignée cellulaire.

Origine de la lignée cellulaire

La lignée cellulaire décrite ici peut être dérivée de toute lignée cellulaire appropriée. Une telle lignée cellulaire est de préférence une lignée cellulaire de mammifère, et encore plus de préférence une lignée cellulaire humaine. Parmi les lignées cellulaires susceptibles d'être utilisées pour générer une lignée décrite ici, on peut notamment citer les lignées usuelles suivantes : SP2/0 ; YB2/0 ; IR983F ; le myélome humain Namalwa ; PERC6 ; les lignées CHO notamment CHO-K-1, CHO Pro-5, CHO dhfr-, Wil-2; Jurkat; Vero; Molt -4; COS-7; 293-HEK; BHK; K6H6; NSO; SP2/0-Ag 14, et P3X63Ag8.653. Si la lignée cellulaire exprime la chaine gamma du récepteur FcεRI humain, la transfection d'un unique vecteur d'expression comprenant une molécule d'acide nucléique codant pour l'antigène CD303 humain est suffisante pour générer une lignée cellulaire telle que décrite ici. Dans le cas contraire, la co-transfection de la lignée cellulaire par un premier vecteur d'expression comprenant une molécule d'acide nucléique codant pour l'antigène CD303 humain et par un autre vecteur d'expression comprenant une molécule d'acide nucléique codant pour la chaine gamma du récepteur FcεRI humain ou la transfection par un vecteur comprenant deux molécules d'acide nucléique codant l'une pour la chaine gamma du récepteur FcεRI humain et l'autre pour l'antigène CD303 humain est nécessaire pour obtenir une lignée cellulaire. L'homme du métier est capable de déterminer quelle lignée cellulaire exprime ou non la chaine gamma du récepteur FcεRI humain pas des expériences de routine (PCR quantitative ou marquage anticorps notamment).

Selon un premier mode de réalisation avantageux de l'invention, la lignée cellulaire selon l'invention peut être une lignée cellulaire de cellules dendritiques plasmacytoïdes humaines (pDC), transfectée de façon stable par un vecteur d'expression comprenant une molécule d'acide nucléique codant pour l'antigène CD303 humain. Les pDC expriment normalement naturellement la chaîne gamma du récepteur FcεRI, et les lignées de pDC humaines ne requièrent donc pas d'être transfectées avec un vecteur d'expression comprenant une molécule d'acide nucléique codant pour la chaine gamma du récepteur FcεRI humain. Sous réserve de confirmation de l'expression de la chaîne gamma du récepteur FcεRI, une simple transfection par un vecteur d'expression comprenant une molécule d'acide nucléique codant pour l'antigène CD303 humain est suffisante.

Avantageusement, la lignée cellulaire selon l'invention est une lignée CAL-1 ou une lignée équivalente transfectée de façon stable par un vecteur comprenant une molécule d'acide nucléique codant pour l'antigène CD303 humain. En effet, la lignée cellulaire CAL-1 (Maeda T et al., Int J Hematol. 2005 Feb ; 81(2) :148-54) est une lignée cellulaire exprimant naturellement la chaîne gamma du récepteur FcεRI, et qui ne requiert donc pas d'être transfectée avec un vecteur d'expression comprenant une molécule d'acide nucléique codant pour la chaine gamma du récepteur FcεRI humain. La lignée cellulaire CAL-1 est dérivée de cellules d'un patient humain souffrant de BPDCN (T et al., Int J Hematol. 2005 Feb ; 81(2) :148-54 et JP 5011520). Les cellules CAL-1 sont définies comme des pDC ayant le phénotype Lin⁻HLA-DR⁺CD11c⁻ CD123⁺CD4⁺CD56⁺. La lignée cellulaire CAL-1 a été déposée selon le Traité de Budapest par l'Université de Nagasaki (1-14 Bunkyo-machi, Nagasaki 852-8521, Japan) auprès du National Institute of Advanced Industrial Science and Technology, International Patent Organism Depository, NITE, (Chuoudai 6, 1-1-1, Higashi, Tsukuba-shi, Ibaraki, au Japon, FERM P-20595 le 15 Juillet 2005, sous le nom de CAL-1), et a été caractérisée en détails dans le brevet JP 5011520.

Toute autre lignée de pDC humaines, telle qu'une autre lignée dérivée de cellules de BPDCN d'un patient humain selon le protocole utilisé pour obtenir la lignée CAL-1 décrit dans Maeda T et al., Int J Hematol. 2005 Feb ; 81(2) :148-54 peut également être utilisée pour obtenir une lignée selon l'invention par transfection stable d'une molécule d'acide nucléique codant pour l'antigène CD303 humain et sélection d'un clone approprié. Le protocole permettant d'obtenir une lignée de pDC humaine à partir de cellules de BPDCN d'un patient humain comprend notamment les étapes suivantes :

- Des cellules malignes primaires de BPDCN sont obtenues à partir du sang périphérique d'un patient diagnostiqué comme souffrant de BPDCN selon la classification de l'OMS. En particulier, les PBMC (cellules mononucléées du sang périphérique) du patient BPDCN sont mises en culture dans un milieu contenant 10% de sérum de veau fœtal (SVF) et 2 mM de L-glutamine, sans IL-2 recombinante, avec changement de milieu deux fois par semaine, et soumis à une culture à long terme (plusieurs semaines ou mois).

- Les cellules survivantes après culture à long terme peuvent ensuite être clonées (par exemple par dilution limite) pour obtenir une lignée cellulaire de longue durée en culture.

De telles lignées obtenues selon le protocole décrit ci-dessus sont considérées comme « équivalentes » à la lignée CAL-1 au sens de la présente invention.

Dans un deuxième mode de réalisation avantageux de l'invention, la lignée cellulaire selon l'invention est une lignée de lymphocyte T humain, transfectée de façon stable par un vecteur d'expression comprenant une première molécule d'acide nucléique codant pour l'antigène CD303 humain et une deuxième molécule d'acide nucléique codant pour la chaine gamma du récepteur FcεRI humain, ou par deux vecteurs d'expressions comprenant chacun une molécule d'acide nucléique codant respectivement pour l'antigène CD303 humain et pour la chaine gamma du récepteur FcεRI humain. En effet, pour les lignées cellulaires n'exprimant pas naturellement la chaîne gamma du récepteur FcεRI, il apparaît comme essentiel de transfecter ces dernières avec une molécule d'acide nucléique codant pour la chaine gamma du récepteur FcεRI humain, pour permettre d'obtenir une lignée exprimant de façon stable l'antigène CD303 à sa surface.

Dans ce cas, la lignée cellulaire selon l'invention peut notamment être une lignée Jurkat (Clone E6-1 : ATCC® TIB -152™, ECACC 88042803 ; Neo Jurkat : ATCC® CRL-2898™ exprimant un gène de résistance à la néomycine), transfectée de façon stable par un vecteur d'expression comprenant une première molécule d'acide nucléique codant pour l'antigène CD303 humain et une deuxième molécule d'acide nucléique codant pour la chaine gamma du récepteur FcεRI humain, ou par deux vecteurs d'expressions comprenant chacun une molécule d'acide nucléique codant respectivement pour l'antigène CD303 humain et pour la chaine gamma du récepteur FcεRI humain. D'autres lignées cellulaires équivalentes accessibles à l'homme du métier peuvent également être utilisées.

## Obtention de la lignée

Tout vecteur d'expression approprié pour une expression dans la lignée cellulaire choisie peut être utilisé, et notamment - dans le cas d'une lignée de cellules de mammifères ou d'une lignée humaine comme la lignée Jurkat ou la lignée CAL-1 de pDC humaines ou une lignée équivalente - tout vecteur d'expression permettant une expression dans les cellules de mammifères, tel que décrit ci-dessous dans la partie concernant les vecteurs ou kits de vecteur.

Le ou les vecteurs d'expression peuvent ensuite être transfectés dans la lignée cellulaire d'intérêt (notamment une lignée de cellules de mammifères ou une lignée humaine comme la lignée Jurkat ou une lignée humaine de pDC) par toute technologie appropriée connue de l'homme du métier, et notamment par nucléofection tel que décrit dans l'Exemple 1, ou par toute autre technologie appropriée, par exemple en utilisant des liposomes, des polymères cationiques, des lipides cationiques, ou par biolistique.

Après transfection d'un ensemble de cellules, les cellules exprimant effectivement l'antigène CD303 à leur surface peuvent être triées par cytométrie en flux. Avantageusement, le vecteur d'expression comprenant une molécule d'acide nucléique codant pour l'antigène CD303 humain comprend également une molécule d'acide nucléique codant pour un marqueur de sélection (par exemple, un gène de résistance à un antibiotique), permettant ainsi de garantir le maintien de l'expression de l'antigène CD303 humain par la lignée cellulaire au cours du temps par culture dans un milieu sélectionnant les cellules exprimant le marqueur de sélection (milieu avec antibiotique dans le cas d'un gène de résistance aux antibiotiques). Après une ou plusieurs périodes de sélection dans un milieu sélectionnant les cellules exprimant le marqueur de sélection et un ou plusieurs tris cellulaires, des clones exprimant l'antigène CD303 humain et la chaine gamma du récepteur FcεRI humain peuvent être obtenus par clonage par dilution limite. Dans ce qui précède, la sélection est fondée uniquement sur l'expression de l'antigène CD303 humain. Cela est lié au fait que la chaine gamma du récepteur FcεRI humain semble nécessaire pour le maintien de l'expression de l'antigène CD303 humain à la surface des cellules. Cependant, une sélection sur la base d'un autre marqueur de sélection présent dans la molécule d'acide nucléique codant pour la chaine gamma du récepteur FcεRI humain peut être ajoutée pour garantir la co-expression des deux protéines dans la lignée cellulaire transfectée.

De façon avantageuse, les transfectants sélectionnés ont une expression de CD303 représentative du CD303 exprimé sur les pDC normales et/ou de patients atteints de maladies auto-immunes ou de leucémie des cellules pDC (BPDCN).

Il est possible d'utiliser une lignée cellulaire déjà transfectée de façon stable par un vecteur d'expression (pcDNA4/TO par exemple) de la chaine gamma du récepteur FcεRI humain appelée lignée « Fc chaine y », et de la transfecter par un autre vecteur d'expression comprenant une molécule d'acide nucléique codant pour l'antigène CD303 humain. Ceci s'applique de toute évidence à la lignée Jurkat dont la forme transfectée chaine FcεRI est appelé « Jurkat Fc chaine y ».

## Nombre de molécules CD303 par cellule

La lignée cellulaire selon l'invention ou décrite ici est transfectée de façon stable par un vecteur d'expression comprenant une molécule d'acide nucléique codant pour l'antigène CD303 humain et présente à sa surface une forte expression de l'antigène CD303 humain. En particulier, la lignée cellulaire selon l'invention présente au moins 10000 molécules du CD303 humain par cellule.

Par « forte expression de l'antigène CD303 humain », on entend que la lignée cellulaire décrite ici, qui a été transfectée de façon stable par un vecteur d'expression comprenant une molécule d'acide nucléique codant pour l'antigène CD303 humain possède un niveau d'expression de l'antigène CD303 humain supérieur à celui de la lignée avant transfection stable par un vecteur d'expression comprenant une molécule d'acide nucléique codant pour l'antigène CD303 humain. Lorsque la lignée cellulaire d'origine n'exprime pas CD303, la lignée transfectée remplit nécessairement ce critère si elle exprime CD303 à sa surface. Lorsque la lignée cellulaire d'origine exprime déjà CD303 (de préférence à un faible niveau), le niveau d'expression de l'antigène CD303 humain de la lignée transfectée décrite ici est avantageusement au moins 10%, au moins 20%, au moins 25%, au moins 30%, au moins 40%, au moins 50%, au moins 60%, au moins 70%, au moins 75%, au moins 80%, au moins 90%, au moins 100% (facteur 2 d'amplification), au moins 125%, au moins 150%, au moins 175%, au moins 200% (facteur 3 d'amplification), au moins 250%, au moins 300% (facteur 4 d'amplification), au moins 350%, au moins 400% (facteur 5 d'amplification), au moins 450%, au moins 500% (facteur 6 d'amplification), au moins 600% (facteur 7 d'amplification), au moins 700% (facteur 8 d'amplification), au moins 800% (facteur 9 d'amplification), au moins 900% (facteur 10 d'amplification), voire au moins 1000% (facteur 11 d'amplification) supérieur à celui de la lignée avant transfection stable par un vecteur d'expression comprenant une molécule d'acide nucléique codant pour l'antigène CD303 humain. Le niveau d'expression peut notamment être exprimé en nombre de molécules de l'antigène CD303 humain par cellule.

Le nombre de molécules d'antigène CD303 humain par cellule peut être déterminé par cytométrie en flux, notamment à l'aide du kit Qifikit commercialisé par Dako, qui permet de déterminer un nombre de sites antigéniques par cellules compris entre 1000 et 1 000 000, et d'anticorps murins anti-(CD303 humain). Le principe du test est le suivant :

- Les cellules sont marquées avec un anticorps monoclonal primaire de souris dirigé contre l'antigène CD303 humain. Dans un tube à essai séparé, les cellules sont marquées avec un anticorps monoclonal primaire contrôle de souris (non pertinent). Ensuite, les cellules et les billes de réglage et d'étalonnage du kit sont étiquetés, en parallèle, avec un anticorps secondaire anti-souris conjugué à la fluorescéine.
- L'anticorps primaire utilisé pour le marquage des cellules est utilisé à concentration saturante. Les conditions de saturation sont déterminées en effectuant des études de titration de chaque anticorps monoclonal primaire de souris en utilisant l'anticorps secondaire anti-souris conjugué à la fluorescéine. L'anticorps primaire peut être de tout isotype d'IgG de souris. Dans ces conditions, le nombre de molécules d'anticorps primaire liés correspond au nombre de sites antigéniques présents à la surface cellulaire.
- L'anticorps secondaire est également utilisé à concentration saturante. En conséquence, la fluorescence est corrélée avec le nombre de molécules d'anticorps primaire liées sur les cellules et sur les billes. Les échantillons sont ensuite analysés dans l'ordre suivant:

  ◦ Tube 1 (billes de réglage) : Cet échantillon est utilisé pour établir la fenêtre d'analyse. Les billes de réglage comprennent un mélange de billes à blanc (aucun site antigénique) et de billes exprimant un haut niveau de sites antigéniques.
  ◦ Tube 2 (billes d'étallonage) : Cet échantillon est utilisé pour la construction de la courbe d'étalonnage (intensité de fluorescence moyenne (MFI)) contre ABC (pour « Antibody-Binding Capacity », soit capacité de liaison de l'anticorps).
  ◦ Les cellules sont ensuite analysées sur le cytomètre de flux et la densité d'antigène sur les cellules (valeur d'ABC) est calculée sur la base de l'équation de la courbe d'étalonnage.

La lignée cellulaire décrite ici présente à sa surface au moins 10000 molécules du CD303 humain par cellule, de préférence entre 10000 et 500000, entre 10000 et 480000, entre 10000 et 460000, entre 10000 et 440000, entre 10000 et 420000, entre 10000 et 400000, entre 10000 et 380000, entre 10000 et 360000, entre 10000 et 340000, entre 10000 et 320000, entre 10000 et 300000, entre 10000 et 280000, entre 10000 et 260000, entre 10000 et 240000, entre 10000 et 220000, entre 10000 et 200000, entre 10000 et 180000, entre 10000 et 160000, entre 10000 et 140000, entre 10000 et 120000, entre 10000 et 100000, entre 10000 et 90000, entre 10000 et 80000, entre 10000 et 70000, entre 10000 et 60000, entre 10000 et 50000, entre 10000 et 40000, entre 10000 et 30000, entre 10000 et 20000, entre 20000 et 500000, entre 20000 et 480000, entre 20000 et 460000, entre 20000 et 440000, entre 20000 et 420000, entre 20000 et 400000, entre 20000 et 380000, entre 20000 et 360000, entre 20000 et 340000, entre 20000 et 320000, entre 20000 et 300000, entre 20000 et 280000, entre 20000 et 260000, entre 20000 et 240000, entre 20000 et 220000, entre 20000 et 200000, entre 20000 et 180000, entre 20000 et 160000, entre 20000 et 140000, entre 20000 et 120000, entre 20000 et 100000, entre 20000 et 90000, entre 20000 et 80000, entre 20000 et 70000, entre 20000 et 60000, entre 20000 et 50000, Réponse notification A94(3) CBE le 12.08.2019 entre 20000 et 40000, entre 20000 et 30000, entre 20000 et 500000, entre 20000 et 480000, entre 20000 et 460000, entre 20000 et 440000, entre 20000 et 420000, entre 20000 et 400000, entre 20000 et 380000, entre 20000 et 360000, entre 20000 et 340000, entre 20000 et 320000, entre 20000 et 300000, entre 20000 et 280000, entre 20000 et 260000, entre 20000 et 240000, entre 20000 et 220000, entre 20000 et 200000, entre 20000 et 180000, entre 20000 et 160000, entre 20000 et 140000, entre 20000 et 120000, entre 20000 et 100000, entre 20000 et 90000, entre 20000 et 80000, entre 20000 et 70000, entre 20000 et 60000, entre 20000 et 50000, entre 20000 et 40000, entre 20000 et 30000, entre 25000 et 500000, entre 25000 et 480000, entre 25000 et 460000, entre 25000 et 440000, entre 25000 et 420000, entre 25000 et 400000, entre 25000 et 380000, entre 25000 et 360000, entre 25000 et 340000, entre 25000 et 320000, entre 25000 et 300000, entre 25000 et 280000, entre 25000 et 260000, entre 25000 et 240000, entre 25000 et 220000, entre 25000 et 200000, entre 25000 et 180000, entre 25000 et 160000, entre 25000 et 140000, entre 25000 et 120000, entre 25000 et 100000, entre 25000 et 90000, entre 25000 et 80000, entre 25000 et 70000, entre 25000 et 60000, entre 25000 et 50000, entre 25000 et 40000, entre 25000 et 30000, entre 30000 et 500000, entre 30000 et 480000, entre 30000 et 460000, entre 30000 et 440000, entre 30000 et 420000, entre 30000 et 400000, entre 30000 et 380000, entre 30000 et 360000, entre 30000 et 340000, entre 30000 et 320000, entre 30000 et 300000, entre 30000 et 280000, entre 30000 et 260000, entre 30000 et 240000, entre 30000 et 220000, entre 30000 et 200000, entre 30000 et 180000, entre 30000 et 160000, entre 30000 et 140000, entre 30000 et 120000, entre 30000 et 100000, entre 30000 et 90000, entre 30000 et 80000, entre 30000 et 70000, entre 30000 et 60000, entre 30000 et 50000, entre 30000 et 40000, entre 40000 et 500000, entre 40000 et 480000, entre 40000 et 460000, entre 40000 et 440000, entre 40000 et 420000, entre 40000 et 400000, entre 40000 et 380000, entre 40000 et 360000, entre 40000 et 340000, entre 40000 et 320000, entre 40000 et 300000, entre 40000 et 280000, entre 40000 et 260000, entre 40000 et 240000, entre 40000 et 220000, entre 40000 et 200000, entre 40000 et 180000, entre 40000 et 160000, entre 40000 et 140000, entre 40000 et 120000, entre 40000 et 100000, entre 40000 et 90000, entre 40000 et 80000, entre 40000 et 70000, entre 40000 et 60000, entre 40000 et 50000, entre 50000 et 500000, entre 50000 et 480000, entre 50000 et 460000, entre 50000 et 440000, entre 50000 et 420000, entre 50000 et 400000, entre 50000 et 380000, entre 50000 et 360000, entre 50000 et 340000, entre 50000 et 320000, entre 50000 et 300000, entre 50000 et 280000, entre 50000 et 260000, entre 50000 et 240000, entre 50000 et 220000, entre 50000 et 200000, entre 50000 et 180000, entre 50000 et 160000, entre 50000 et 140000, entre 50000 et 120000, entre 50000 et 100000, entre 50000 et 90000, entre 50000 et 80000, entre 50000 et 70000, entre 50000 et 60000, entre 60000 et 500000, entre 60000 et 480000, entre 60000 et 460000, entre 60000 et 440000, entre 60000 et 420000, entre 60000 et 400000, entre 60000 et 380000, entre 60000 et 360000, entre 60000 et 340000, entre 60000 et 320000, entre 60000 et 300000, entre 60000 et 280000, entre 60000 et 260000, entre 60000 et 240000, entre 60000 et 220000, entre 60000 et 200000, entre 60000 et 180000, entre 60000 et 160000, entre 60000 et 140000, entre 60000 et 120000, entre 60000 et 100000, entre 60000 et 90000, entre 60000 et 80000, entre 60000 et 70000, entre 70000 et 500000, entre 70000 et 480000, entre 70000 et 460000, entre 70000 et 440000, entre 70000 et 420000, entre 70000 et 400000, entre 70000 et 380000, entre 70000 et 360000, entre 70000 et 340000, entre 70000 et 320000, entre 70000 et 300000, entre 70000 et 280000, entre 70000 et 260000, entre 70000 et 240000, entre 70000 et 220000, entre 70000 et 200000, entre 70000 et 180000, entre 70000 et 160000, entre 70000 et 140000, entre 70000 et 120000, entre 70000 et 100000, entre 70000 et 90000, entre 70000 et 80000, entre 80000 et 500000, entre 80000 et 480000, entre 80000 et 460000, entre 80000 et 440000, entre 80000 et 420000, entre 80000 et 400000, entre 80000 et 380000, entre 80000 et 360000, entre 80000 et 340000, entre 80000 et 320000, entre 80000 et 300000, entre 80000 et 280000, entre 80000 et 260000, entre 80000 et 240000, entre 80000 et 220000, entre 80000 et 200000, entre 80000 et 180000, entre 80000 et 160000, entre 80000 et 140000, entre 80000 et 120000, entre 80000 et 100000, entre 80000 et 90000, entre 90000 et 500000, entre 90000 et 480000, entre 90000 et 460000, entre 90000 et 440000, entre 90000 et 420000, entre 90000 et 400000, entre 90000 et 380000, entre 90000 et 360000, entre 90000 et 340000, entre 90000 et 320000, entre 90000 et 300000, entre 90000 et 280000, entre 90000 et 260000, entre 90000 et 240000, entre 90000 et 220000, entre 90000 et 200000, entre 90000 et 180000, entre 90000 et 160000, entre 90000 et 140000, entre 90000 et 120000, entre 90000 et 100000, entre 100000 et 500000, entre 100000 et 480000, entre 100000 et 460000, entre 100000 et 440000, entre 100000 et 420000, entre 100000 et 400000, entre 100000 et 380000, entre 100000 et 360000, entre 100000 et 340000, entre 100000 et 320000, entre 100000 et 300000, entre 100000 et 280000, entre 100000 et 260000, entre 100000 et 240000, entre 100000 et 220000, entre 100000 et 200000, entre 100000 et 180000, entre 100000 et 160000, entre 100000 et 140000, entre 100000 et 120000, entre 120000 et 500000, entre 120000 et 480000, entre 120000 et 460000, entre 120000 et 440000, entre 120000 et 420000, entre 120000 et 400000, entre 120000 et 380000, entre 120000 et 360000, entre 120000 et 340000, entre 120000 et 320000, entre 120000 et 300000, entre 120000 et 280000, entre 120000 et 260000, entre 120000 et 240000, entre 120000 et 220000, entre 120000 et 200000, entre 120000 et 180000, entre 120000 et 160000, entre 120000 et 140000, entre 140000 et 500000, entre 140000 et 480000, entre 140000 et 460000, entre 140000 et 440000, entre 140000 et 420000, entre 140000 et 400000, entre 140000 et 380000, entre 140000 et 360000, entre 140000 et 340000, entre 140000 et 320000, entre 140000 et 300000, entre 140000 et 280000, entre 140000 et 260000, entre 140000 et 240000, entre 140000 et 220000, entre 140000 et 200000, entre 140000 et 180000, entre 140000 et 160000, entre 240000 et 500000, entre 160000 et 480000, entre 160000 et 460000, entre 160000 et 440000, entre 160000 et 420000, entre 160000 et 400000, entre 160000 et 380000, entre 160000 et 360000, entre 160000 et 340000, entre 160000 et 320000, entre 160000 et 300000, entre 160000 et 280000, entre 160000 et 260000, entre 160000 et 240000, entre 160000 et 220000, entre 160000 et 200000, entre 160000 et 180000, entre 180000 et 500000, entre 180000 et 480000, entre 180000 et 460000, entre 180000 et 440000, entre 180000 et 420000, entre 180000 et 400000, entre 180000 et 380000, entre 180000 et 360000, entre 180000 et 340000, entre 180000 et 320000, entre 180000 et 300000, entre 180000 et 280000, entre 180000 et 260000, entre 180000 et 240000, entre 180000 et 220000, entre 180000 et 200000, entre 200000 et 500000, entre 200000 et 480000, entre 200000 et 460000, entre 200000 et 440000, entre 200000 et 420000, entre 200000 et 400000, entre 200000 et 380000, entre 200000 et 360000, entre 200000 et 340000, entre 200000 et 320000, entre 200000 et 300000, entre 200000 et 280000, entre 200000 et 260000, entre 200000 et 240000, entre 200000 et 220000, entre 220000 et 500000, entre 220000 et 480000, entre 220000 et 460000, entre 220000 et 440000, entre 220000 et 420000, entre 220000 et 400000, entre 220000 et 380000, entre 220000 et 360000, entre 220000 et 340000, entre 220000 et 320000, entre 220000 et 300000, entre 220000 et 280000, entre 220000 et 260000, entre 220000 et 240000, entre 240000 et 500000, entre 240000 et 480000, entre 240000 et 460000, entre 240000 et 440000, entre 240000 et 420000, entre 240000 et 400000, entre 240000 et 380000, entre 240000 et 360000, entre 240000 et 340000, entre 240000 et 320000, entre 240000 et 300000, entre 240000 et 280000, entre 240000 et 260000, entre 260000 et 500000, entre 260000 et 480000, entre 260000 et 460000, entre 260000 et 440000, entre 260000 et 420000, entre 260000 et 400000, entre 260000 et 380000, entre 260000 et 360000, entre 260000 et 340000, entre 260000 et 320000, entre 260000 et 300000, entre 260000 et 280000, entre 280000 et 500000, entre 280000 et 480000, entre 280000 et 460000, entre 280000 et 440000, entre 280000 et 420000, entre 280000 et 400000, entre 280000 et 380000, entre 280000 et 360000, entre 280000 et 340000, entre 280000 et 320000, entre 280000 et 300000, entre 300000 et 500000, entre 300000 et 480000, entre 300000 et 460000, entre 300000 et 440000, entre 300000 et 420000, entre 300000 et 400000, entre 300000 et 380000, entre 300000 et 360000, entre 300000 et 340000, entre 300000 et 320000, entre 320000 et 500000, entre 320000 et 480000, entre 320000 et 460000, entre 320000 et 440000, entre 320000 et 420000, entre 320000 et 400000, entre 320000 et 380000, entre 320000 et 360000, entre 320000 et 340000, entre 340000 et 500000, entre 340000 et 480000, entre 340000 et 460000, entre 340000 et 440000, entre 340000 et 420000, entre 340000 et 400000, entre 340000 et 380000, entre 340000 et 360000, entre 360000 et 500000, entre 360000 et 480000, entre 360000 et 460000, entre 360000 et 440000, entre 360000 et 420000, entre 360000 et 400000, entre 360000 et 380000, entre 380000 et 500000, entre 380000 et 480000, entre 380000 et 460000, entre 380000 et 440000, entre 380000 et 420000, entre 380000 et 400000, entre 400000 et 500000, entre 400000 et 480000, entre 400000 et 460000, entre 400000 et 440000, entre 400000 et 420000, entre 420000 et 500000, entre 420000 et 480000, entre 420000 et 460000, entre 420000 et 440000, entre 440000 et 500000, entre 440000 et 480000, entre 440000 et 460000, entre 460000 et 500000, entre 460000 et 480000, ou entre 480000 et 500000 molécules du CD303 humain par cellule.

Selon un mode de réalisation préféré, la lignée cellulaire selon l'invention présente à sa surface entre 20000 et 60000, préférentiellement entre 25000 et 50000 molécules du CD303 humain par cellule. En effet, de tels nombres de molécules de CD303 humain par cellule permettent de discriminer efficacement, et de façon reproductible, des anticorps dirigés contre l'antigène CD303 humain.

### Vecteur double ou kit de deux vecteurs

Un vecteur d'expression comprenant une première molécule d'acide nucléique codant pour l'antigène CD303 humain et une deuxième molécule d'acide nucléique codant pour la chaine gamma du récepteur FcɛRI humain est également décrit. Alternativement, les molécules d'acides nucléiques codant respectivement pour l'antigène CD303 humain et pour la chaine gamma du récepteur FcɛRI humain sont présentes sur deux vecteurs d'expressions indépendants.

Un kit de transfection comprenant un vecteur d'expression comprenant une première molécule d'acide nucléique codant pour l'antigène CD303 humain et une deuxième molécule d'acide nucléique codant pour la chaine gamma du récepteur FcɛRI humain, ou deux vecteurs d'expression comprenant chacun une molécule d'acide nucléique codant respectivement pour l'antigène CD303 humain et pour la chaine gamma du récepteur FcɛRI humain est également décrit.

Chaque vecteur décrit ici ou inclut dans un kit de transfection décrit ici comprend les éléments nécessaires à l'expression de ladite molécule d'acide nucléique, et notamment un promoteur, un codon d'initiation de la transcription, des séquences de terminaison, et des séquences de régulation de la transcription appropriées. Ces éléments varient en fonction de l'hôte servant pour l'expression et sont choisis aisément par l'homme du métier au vu de ses connaissances générales. Le vecteur peut notamment être plasmidique ou viral.

Dans le cas d'une lignée de cellules de mammifères ou d'une lignée humaine comme la lignée Jurkat ou une lignée humaine de pDC telle que CAL-1, tout vecteur d'expression permettant une expression dans les cellules de mammifères pourra être utilisé.

Des exemples de tels vecteurs incluent le vecteur pcDNA4/TO (Invitrogen) utilisé pour exprimer la chaine gamma du

récepteur FcɛRI humain dans une lignée Jurkat E6-1 (ATCC® TIB -152™, ECACC 88042803), ou le vecteur pcDNA3.1 (-) utilisé dans l'Exemple 1 pour exprimer l'antigène CD303 humain.

### *Utilisations*

Selon un autre objet, l'invention concerne l'utilisation de la lignée cellulaire selon l'invention pour caractériser une activité fonctionnelle d'un anticorps dirigé contre l'antigène CD303 humain, préférentiellement une activité de type ADCC, CDC, sécrétion de cytokines, apoptose, et phagocytose ou pour comparer l'affinité de liaison de plusieurs anticorps anti-CD303 à l'antigène CD303 humain ou les épitopes de l'antigène CD303 humain reconnus par plusieurs anticorps anti-CD303.

Selon un autre objet, l'invention concerne l'utilisation de la lignée cellulaire selon l'invention pour caractériser les capacités fonctionnelles (notamment les capacités de type ADCC, CDC, sécrétion de cytokines, apoptose, et phagocytose) des cellules effectrices d'un sujet humain, en particulier un sujet humain souffrant de BPDCN ou d'une maladie autoimmune.

Caractérisation d'anticorps anti-CD303

*Anticorps anti-CD303 susceptibles d'être caractérisés à l'aide des cellules selon l'invention*

Par « anticorps », on entend une molécule comprenant au moins un domaine de liaison à un antigène donné et un domaine constant comprenant un fragment Fc capable de se lier aux récepteurs FcR. Chez la plupart des mammifères, comme l'homme et la souris, un anticorps est composé de 4 chaînes polypeptidiques : 2 chaînes lourdes et 2 chaînes légères reliées entre elles par un nombre variable de ponts disulfures assurant une flexibilité à la molécule. Chaque chaîne légère est constituée d'un domaine constant (CL) et d'un domaine variable (VL); les chaînes lourdes étant composées d'un domaine variable (VH) et de 3 ou 4 domaines constants (CH1 à CH3 ou CH1 à CH4) selon l'isotype de l'anticorps. Les domaines variables sont impliqués dans la reconnaissance de l'antigène, tandis que les domaines constants sont impliqués dans les propriétés biologiques, pharmacocinétiques et effectrices, de l'anticorps. La région variable diffère d'un anticorps à un autre. En effet, les gènes codant pour les chaînes lourdes et légères des anticorps sont générés par recombinaison de respectivement trois et deux segments de gènes distincts appelés VH, DH et JH-CH pour la chaîne lourde et VL et JL-CL pour la chaîne légère. Les segments CH et CL ne participent pas à la recombinaison et forment les régions constantes des chaînes lourdes et légères respectivement. Les recombinaisons des segments VH-DH-JH et VL-JL forment les régions variables des chaînes lourdes et légères respectivement. Les régions VH et VL possèdent chacune 3 zones hyper variables ou régions déterminant la complémentarité (CDR), appelées CDR1, CDR2 et CDR3, la région CDR3 étant la plus variable, puisqu'elle se situe au niveau de la zone de recombinaison. Ces trois régions CDR, et particulièrement la région CDR3, se trouvent dans la partie de l'anticorps qui sera en contact avec l'antigène et sont donc très importantes pour la reconnaissance de l'antigène. Ainsi, les anticorps conservant les trois régions CDR et chacune des chaînes lourde et légère d'un anticorps conservent en grande majorité la spécificité antigénique de l'anticorps d'origine. Dans un certain nombre de cas, un anticorps ne conservant que l'un des CDR, et notamment le CDR3, conserve également la spécificité de l'anticorps d'origine. Les régions CDR1, CDR2 et CDR3 sont chacune précédées des régions FR1, FR2 et FR3 respectivement, correspondant aux régions charpentes (framework région FR) qui varient le moins d'un segment VH ou VL à un autre. La région CDR3 est également suivie d'une région charpente FR4.

Dans le cadre de l'invention, tout anticorps dirigé contre l'antigène CD303 humain peut être caractérisé à l'aide des cellules selon l'invention, quelles que soient les séquences primaires de ses chaines lourdes et légères, et notamment quelles que soient les séquences des CDR1, CDR2, et CDR3 des chaines lourdes et légères.

Contrairement aux domaines variables dont la séquence varie fortement d'un anticorps à un autre, les domaines constants sont caractérisés par une séquence en acides aminés très proche d'un anticorps à l'autre, caractéristique de l'espèce et de l'isotype, avec éventuellement quelques mutations somatiques. Le fragment Fc est naturellement composé de la région constante de la chaine lourde à l'exclusion du domaine CH1, c'est-à-dire de la région charnière inférieure et des domaines constants CH2 et CH3 ou CH2 à CH4 (selon l'isotype). Chez les IgG1 humaines, le fragment Fc complet est composé de la partie C-terminale de la chaine lourde à partir du résidu cystéine en position 226 (C226), la numérotation des résidus d'acides aminés dans le fragment Fc étant dans toute la présente description celle de l'index EU décrit dans Edelman et al-1969 et Kabat et al-1991. Les fragments Fc correspondants d'autres types d'immunoglobulines peuvent être identifiés aisément par l'homme du métier par des alignements de séquences.

Le fragment Fc est glycosylé au niveau du domaine CH2 avec la présence, sur chacune des 2 chaînes lourdes, d'un N-glycanne lié au résidu asparagine en position 297 (Asn 297).

La glycosylation est connue pour influencer les fonctions effectrices de l'anticorps, telles que ses activités ADCC et CDC. Notamment, il est aujourd'hui connu que la teneur en fucose d'une composition d'anticorps joue un rôle crucial dans la capacité de cette composition à induire une forte réponse ADCC via le récepteur FcγRIII (WO01/77181, Shields et al-2002 ; Shinkawa et al-2003). L'anticorps à caractériser sur le plan fonctionnel dans le cadre de l'invention peut posséder une faible teneur en fucose, notamment inférieure ou égale à 65%. Par « teneur en fucose », on entend le pourcentage de formes fucosylées au sein des N-glycannes attachés au résidu Asn297 du fragment Fc de chaque chaine lourde de chaque anticorps. Par « faible teneur en fucose », on entend une teneur en fucose inférieure ou égale à 65%, voire plus faible, mais pas nécessairement nulle. La teneur en fucose peut par exemple être comprise entre 5 et 65% ou entre 5 et 50%, ou entre 10 et 50%. L'anticorps, fragment fonctionnel ou dérivé de celui-ci peut par ailleurs posséder différents types de glycosylation (N-glycannes de type oligomannose ou complexes biantennés, proportion variable de résidus N-acétylglucosamine (GlcNAc) intercalaires ou de résidus galactose dans le cas des N-glycannes de type complexes biantennés), à condition de posséder un faible teneur en fucose. Des anticorps possédant des N-glycannes faiblement fucosylés ont été notamment obtenus par :

- Production dans YB2/0 (voir EP1176195A1, WO01/77181, Shinkawa et al-2003) CHO Lec13 (voir Shields et al-2002), EB66® (Olivier et al-2010), les lignées d'hépatome de rat rat H4-II-E (DSM ACC3129), H4-II-Es (DSM ACC3130) (voir WO2012/041768), et les lignées humaines NM-H9D8 (DSM ACC2806), NM-H9D8-E6 (DSM ACC 2807), et NM H9D8-E6Q12 (DSM ACC 2856) (voir WO2008/028686).
- Production dans une lignée CHO sauvage en présence de petits ARNs interférents dirigés contre FUT8 (Mori et al-2004, Suzuki et al-2007, Cardarelli et al-2009, Cardarelli et al-2010, Herbst et al-2010), ou GMD (gène codant pour le transporteur du GDP-fucose dans le Golgi, voir Imai-Nishiya et al-2007)
- Production dans une lignée CHO dont les deux allèles du gène FUT8 codant pour la 1,6-fucosyltransférase ont été délétés (Yamane-Ohnuki et al-2004), ou dont les deux allèles du gène GMD codant pour le transporteur du GDP-fucose dans le Golgi ont été délétés (Kanda et al-2007),
- Production dans une lignée CHO dans laquelle le gène codant pour l'enzyme GnTIII (β(1,4)-N-acétylglucosaminetransférase III) a été surexprimé par transgénèse (Umana et al-1999). Outre une faible fucosylation, les N-glycannes obtenus sont caractérisés par une forte teneur en GlcNAc intercalaire.
- Production dans des plantes (N. benthamiana) transgéniques, avec une forte diminution des teneurs en résidus β1,2-xylose et α1,3-fucose grâce à l'utilisation de petits ARNs interférents (Forthal et al-2010).

En sus ou alternativement à une faible teneur en fucose, l'anticorps à caractériser sur le plan fonctionnel dans le cadre de l'invention peut posséder une forte teneur en galactose.

Par « teneur en galactose » ou « taux de galactosylation » de l'anticorps, on entend un pourcentage calculé à partir d'un chromatogramme d'analyse des N-glycannes libérés de l'anticorps, selon la formule suivante :

$$\text{teneur en galactose} = \frac{\sum_{i=1}^{n}(\text{nombre de Gal}) \times (\% \text{ surface relative})}{\sum_{i=1}^{n}(\text{nombre de A}) \times (\% \text{ surface relative})} \times 100$$

dans laquelle :

- « n » représente le nombre de pics N-glycannes analysés sur un chromatogramme, par exemple d'un spectre de chromatographie en phase liquide à haute performance en phase normale (NP HPLC),
- « nombre de Gal » représente le nombre de galactoses sur l'antenne du glycanne correspondant au pic,
- « nombre de A » représente le nombre d'antennes N-acétyl-glucosamine de la forme glycannique correspondant au pic, et
- « % surface relative » correspond au pourcentage de l'aire sous le pic correspondant.

Par « forte teneur en galactose », on entend une teneur en galactose supérieure ou égale à 30%.

Les domaines de liaison suivants, situés dans le Fc, sont importants pour les propriétés biologiques de l'anticorps :

- domaine de liaison au récepteur FcRn, impliqué dans les propriétés pharmacocinétiques (demi-vie *in vivo*) de l'anticorps :
Différentes données suggèrent que certains résidus situés à l'interface des domaines CH2 et CH3 sont impliqués dans la liaison au récepteur FcRn.
- domaine de liaison à la protéine du complément C1q, impliqué dans la réponse CDC (pour « cytotoxicité dépendante du complément ») : situé dans le domaine CH2;

- domaine de liaison aux récepteurs FcR, impliqué dans les réponses de type phagocytose ou ADCC (pour « cytotoxicité cellulaire dépendante de l'anticorps ») : situé dans le domaine CH2.

Au sens de l'invention, le fragment Fc d'un anticorps peut être naturel, tel que défini ci-dessus, ou bien avoir été modifié de diverses façons, à condition de comprendre un domaine de liaison aux récepteurs FcR (récepteurs FcγR pour les IgG) fonctionnel, et de préférence un domaine de liaison au récepteur FcRn fonctionnel. Les modifications peuvent inclure la délétion de certaines parties du fragment Fc, pourvu que celui-ci contienne un domaine de liaison aux récepteurs FcR (récepteurs FcγR pour les IgG) fonctionnel, et de préférence un domaine de liaison au récepteur FcRn fonctionnel.

Les modifications peuvent également inclure différentes substitutions d'acides aminés susceptibles d'affecter les propriétés biologiques de l'anticorps, pourvu que celui-ci contienne un domaine de liaison aux récepteurs FcR fonctionnel, et de préférence un domaine de liaison au récepteur FcRn fonctionnel. En particulier, lorsque l'anticorps est un IgG, il peut comprendre des mutations destinées à augmenter la liaison au récepteur FcγRIIIa (CD16a), telles que décrites dans WO00/42072, Shields et al-2001, Lazar et al-2006, WO2004/029207, WO2004/063351, WO2004/074455. Des mutations permettant d'augmenter la liaison au récepteur FcRn et donc la demi-vie *in vivo* peuvent également être présentes, comme décrit par exemple dans Shields et al-2001, Dall'Acqua et al-2002, Hinton et al-2004, Dall'Acqua et al-2006(a), WO00/42072, WO02/060919, WO2010/045193, ou WO2010/106180. D'autres mutations, comme celles permettant de diminuer ou d'augmenter la liaison aux protéines du complément et donc la réponse CDC, peuvent ou non être présentes (voir WO99/51642, WO2004/074455, Idusogie et al-2001, Dall'Acqua et al-2006(b), et Moore et al-2010).

Dans le cadre de l'invention, les anticorps à caractériser peuvent être des anticorps de tout type d'animal (notamment des anticorps murins), chimériques, humanisés, ou humains. Notamment, pour la caractérisation de l'affinité pour l'antigène, tout type d'anticorps (murins ou autres animaux, chimériques, humanisés, ou humains) peut être utilisé. Pour la caractérisation d'une activité fonctionnelle, l'anticorps à caractérisé est de préférence un anticorps chimérique, humanisé, ou humain, permettant ainsi de tester les fonctions de l'anticorps dans un cadre humain (cellules effectrices humaines, sérum humain...).

Par anticorps « chimérique », on entend désigner un anticorps qui contient une région variable (chaîne légère et chaîne lourde) naturelle dérivée d'un anticorps d'une espèce donnée en association avec les régions constantes de chaîne légère et chaîne lourde d'un anticorps d'une espèce hétérologue à ladite espèce donnée. Avantageusement, si la composition d'anticorps monoclonal pour son utilisation en tant que médicament comprend un anticorps monoclonal chimérique, celui-ci comprend des régions constantes humaines. Partant d'un anticorps non humain, un anticorps chimérique peut être préparé en utilisant les techniques de recombinaison génétique bien connues de l'homme du métier. Par exemple, l'anticorps chimérique pourra être réalisé en clonant pour la chaine lourde et la chaine légère un ADN recombinant comportant un promoteur et une séquence codant pour la région variable de l'anticorps non humain, et une séquence codant pour la région constante d'un anticorps humain. Pour les méthodes de préparation d'anticorps chimériques, on pourra par exemple se référer au document Verhoeyn et al-1988.

Par anticorps « humanisé », on entend désigner un anticorps qui contient des régions CDRs dérivées d'un anticorps d'origine non humaine, les autres parties de la molécule d'anticorps étant dérivées d'un (ou de plusieurs) anticorps humains. En outre, certains des résidus des segments du squelette (dénommés FR) peuvent être modifiés pour conserver l'affinité de liaison (Jones et al-1986 ; Verhoeyen et al- 1988 ; Riechmann et al-1988). Les anticorps humanisés peuvent être préparés par des techniques connues de l'homme de l'art telles les technologies de « CDR grafting », de « resurfacing », de SuperHumanisation, de « Human string content », de « FR libraries », de « Guided selection », de « FR shuffling » et de « Humaneering », comme résumé dans la revue de Almagro et al-2008.

Dans le cadre de l'invention, les anticorps à caractériser sont avantageusement des anticorps monoclonaux. Par « anticorps monoclonal » ou « composition d'anticorps monoclonal », on entend une composition comprenant des molécules d'anticorps possédant une spécificité antigénique identique et unique. Les molécules d'anticorps présentes dans la composition sont susceptibles de varier au niveau de leurs modifications post-traductionnelles, et notamment au niveau de leurs structures de glycosylation ou de leur point isoélectrique, mais ont toutes été codées par les mêmes séquences de chaines lourde et légère et ont donc, avant toute modification post-traductionnelle, la même séquence protéique. Certaines différences de séquences protéique, liées à des modifications post-traductionnelles (comme par exemple le clivage de la lysine C-terminale de la chaine lourde, la déamidation de résidus asparagine et/ou l'isomérisation de résidus aspartate), peuvent néanmoins exister entre les différentes molécules d'anticorps présentes dans la composition.

Dans le cadre de l'invention, les anticorps à caractériser peuvent être de plusieurs isotypes. En effet, les anticorps peuvent être de plusieurs isotypes, en fonction de la nature de leur région constante : les régions constantes γ, α, μ, ε et δ correspondent respectivement aux immunoglobulines IgG, IgA, IgM, IgE et IgD. Avantageusement, l'anticorps monoclonal à caractériser sur le plan fonctionnel dans le cadre de l'invention est d'isotype IgG. En effet, cet isotype montre une capacité à engendrer une activité ADCC (« Antibody-Dependent Cellular Cytotoxicity », soit Cytotoxicité cellulaire dépendante de l'anticorps) chez le plus grand nombre d'individus (humains). Les régions constantes γ comprennent plusieurs sous-types : γ1, γ2, γ3, ces trois types de régions constantes présentent la particularité de fixer le complément humain, et γ4, créant ainsi les sous-isotypes IgG1, IgG2, IgG3, et IgG4. Avantageusement, l'anticorps monoclonal à caractériser sur le plan fonctionnel dans le cadre de l'invention est d'isotype IgG1 ou IgG3, de préférence IgG1.

L'anticorps à caractériser sur le plan fonctionnel dans le cadre de l'invention peut être produit à partir de toute cellule hôte, de tout animal non-humain transgénique ou de toute plante transgénique approprié.

Les anticorps dirigés contre l'antigène CD303 humain exprimé par la lignée cellulaire selon l'invention qui sont à caractériser peuvent être destinés au traitement ou à la prévention des tumeurs hématopoïétiques exprimant l'antigène CD303. Il s'agit notamment des néoplasmes de cellules dendritiques plasmacytoïdes blastiques (BPDCN), de phénotype CD4+, CD11c-, Lin-, CD303+, CD304+, CD56+.

Ces anticorps peuvent également être destinés au traitement ou à la prévention des maladies inflammatoires, notamment des maladies autoimmunes, des maladies impliquant les pDC, et plus particulièrement des maladies impliquant une sécrétion d'IFN-α par les pDC, telle que la dermatite atopique, la dermatite de contact, le psoriasis, le lupus érythémateux disséminé, la dermatomyosite, le syndrome de Sjögren, le diabète de type 1b, la thrombocytopénie (ou thrombopénie) autoimmune (notamment le purpura thrombopénique idiopathique ou PTI), la sclérodermie systémique (encore appelée sclérodermie systémique progressive ou sclérose systémique), la polyarthrite rhumatoïde.

Dans le cadre de la présente invention, les lignées cellulaires selon l'invention permettent donc d'évaluer l'activité fonctionnelle d'un anticorps dirigé contre l'antigène CD303 humain ou de comparer l'affinité de plusieurs anticorps anti-CD303 pour l'antigène CD303 humain ou les épitopes de l'antigène CD303 humain reconnus par plusieurs anticorps anti-CD303.

*Caractérisation de l'activité ADCC*

Dans un premier mode de réalisation, l'utilisation des lignées cellulaires selon l'invention vise à caractériser l'activité ADCC de l'anticorps anti-CD303 humain. Ainsi, l'invention concerne l'utilisation de la lignée cellulaire selon l'invention pour caractériser l'activité ADCC d'un anticorps dirigé contre l'antigène CD303 humain.

Par « activité ADCC » (ADCC étant l'abréviation de « antibody dépendent cellular cytotoxicity », soit « cytotoxicité cellulaire dépendante de l'anticorps »), on entend la capacité de l'anticorps à induire la mort de cellules cibles (ici, les cellules de la lignée cellulaire selon l'invention) en présence de cellules effectrices du système immunitaire, par sa liaison d'une part aux cellules cibles par l'intermédiaire de ses parties variables se liant à l'antigène situé à la surface des cellules cibles, et d'autre part aux cellules effectrices par l'intermédiaire de la liaison de son fragment Fc à un récepteur Fc (FcR, FcγR dans le cas des IgG) situé à la surface des cellules effectrices. La mesure de l'activité ADCC d'un anticorps est réalisée par des techniques conventionnelles. Notamment, dans un mode de réalisation, l'invention concerne un procédé de test de l'activité ADCC d'un anticorps dirigé contre l'antigène CD303 humain, comprenant les étapes suivantes :

a) mise en contact de la lignée cellulaire selon l'invention avec un anticorps dirigé contre l'antigène CD303 humain et des cellules effectrices ;

b) incubation pendant une durée appropriée pour permettre la lyse des cellules de la lignée cellulaire selon l'invention de l'étape a); et

c) mesure du taux de lyse des cellules de la lignée cellulaire selon l'invention de l'étape a).

A l'étape a), la lignée cellulaire selon l'invention (cellules cibles) est mise en contact avec un anticorps dirigé contre l'antigène CD303 humain à caractériser (tel que décrit ci-dessus) et des cellules effectrices.

Dans le cadre de la caractérisation de l'activité ADCC, les cellules effectrices doivent être capables d'induire une lyse des cellules cibles. Il peut s'agir des monocytes, macrophages, lymphocytes T ou cellules NK. Toutes ces cellules sont présentes dans les PBMC (abréviation de « Peripheral blood mononuclear cells », soit cellules mononucléées du sang périphérique), et des PBMC peuvent donc être utilisés. Les cellules effectrices utilisées sont compatibles avec les anticorps à caractériser, et sont donc de la même espèce que le fragment Fc de l'anticorps anti-CD303 humain à caractériser.

Dans le cas des IgG, l'ADCC via le récepteur FcγRIIIa est connue pour être celle essentielle pour une bonne activité in vivo, et le test ADCC selon l'invention mesure donc avantageusement spécifiquement l'ADCC via le récepteur FcγRIIIa. Les cellules effectrices expriment pour la plupart deux récepteurs FcγR capables d'induire une réponse ADCC : le récepteur de forte affinité pour le fragment Fc des IgG FcγRI et le récepteur de faible affinité pour le fragment Fc des IgG FcγRIIIa. Les cellules NK sont une exception et n'expriment que le récepteur FcγRIIIa. Ainsi, pour mesurer spécifiquement l'ADCC via le récepteur FcγRIIIa, il est possible d'utiliser des cellules NK purifiées comme cellules effectrices. De manière alternative, il est également possible d'utiliser des cellules effectrices exprimant les deux récepteurs FcγRI et FcγRIIIa (ou un mélange de cellules exprimant pour certaines les deux récepteurs FcγRI et FcγRIIIa et pour d'autres uniquement le récepteur FcγRIIIa) et de saturer les récepteurs FcγRI ayant une forte affinité pour le fragment Fc des IgG par ajout dans le milieu réactionnel d'IgG polyvalentes, c'est-à-dire d'IgG polyclonales de multiples spécificités antigéniques, purifiées à partir de plasma. Lorsque des cellules effectrices humaines sont utilisées, on peut notamment utiliser des IVIG, ou immunoglobulines humaines normales, IgG polyvalentes médicament utilisées dans le traitement de certaines pathologies autoimmunes.

Les cellules cibles (cellules qui expriment à leur surface l'antigène CD303 humain reconnu par l'anticorps à tester, ici les cellules de la lignée cellulaire selon l'invention) peuvent avoir préalablement été marquées de façon intracellulaire par un marqueur permettant à l'étape c) de mesurer le nombre de cellules cibles effectivement lysées. Tout marqueur approprié, tel qu'un marqueur radioactif, fluorescent, luminescent, ou chromogénique peut être utilisé. De manière alternative, les cellules cibles peuvent ne pas être marquées préalablement. On peut alors utiliser le dosage d'une protéine présente dans les cellules cibles (telle que l'enzyme LDH présente dans tous types de cellules) pour mesurer le nombre de cellules lysées à l'étape c).

Les cellules cibles (cellules qui expriment à leur surface l'antigène CD303 humain reconnu par l'anticorps à tester, ici les cellules de la lignée cellulaire selon l'invention) et les cellules effectrices sont présentes dans un ratio effecteur/cible (E/C) proche des conditions physiologiques *in vivo.*

On réalise généralement une courbe dose/réponse d'ADCC. Pour cela, des quantités croissantes d'anticorps sont mises en contact avec les cellules cibles et les cellules effectrices dans des puits séparés et l'ADCC mesurée à différentes concentrations d'anticorps. Chaque point est généralement réalisé en plusieurs exemplaires (duplicats ou triplicats notamment).

Des contrôles négatifs sont également réalisés, généralement :

- lyse ou relargage spontané (SR), obtenu en mettant en contact les cellules cibles uniquement avec les anticorps (sans cellules effectrices),
- la cytotoxicité naturelle (NC) des cellules effectrices, obtenue en mettant en contact les cellules cibles uniquement avec les cellules effectrices (sans anticorps).

Un contrôle positif de lyse maximale, obtenu en lysant les cellules cibles avec un produit chimique, peut également être utilisé.

A l'étape b), on réalise une incubation (généralement à 37°C) pendant une durée appropriée pour permettre la lyse des cellules cibles (cellules de la lignée cellulaire selon l'invention). La durée d'incubation peut varier entre quelques heures et environ une journée, notamment entre 1 et 24 heures, en particulier pendant 4 à 24 heures. L'homme du métier sait déterminer une durée optimale en fonction des conditions particulières du test.

Enfin, à l'étape c), on mesure le taux de lyse des cellules cibles (cellules de la lignée cellulaire selon l'invention). Ceci est généralement réalisé en centrifugeant les plaques utilisées pour la mise en contact et l'incubation, en récupérant le surnageant et en mesurant soit la quantité de marqueur intracellulaire des cellules cibles préalablement marquées relarguée dans le milieu, soit la quantité ou l'activité d'une protéine initialement présente dans les cellules cibles et relarguée dans le milieu suite à la lyse des cellules cibles.

Si les contrôles mentionnés ci-dessus sont utilisés, le pourcentage de lyse peut alors être calculé selon l'une des formules suivantes :

$$\% \text{ lyse} = [(ER - SR) / (100 - SR)] - [(NC - SR) / (100 - SR)]$$

ou

$$\% \text{ de lyse} = ER - NC - SR$$

Où SR et NC sont tels que définis ci-dessus, et ER correspond à la lyse obtenue pour l'échantillon test.

Les résultats (% lyse) sont exprimés en fonction du facteur de dilution de l'anticorps. Pour chaque anticorps, une valeur d'« activité 50% » ou « EC50 »peut être calculée, qui correspond au facteur de dilution de l'anticorps nécessaire pour induire 50% de la valeur plateau obtenue pour cet anticorps.

Un exemple précis de test ADCC via le récepteur FcγRIIIa selon l'invention est le suivant :

Des cellules Jurkat chaine Fc Gamma-CD303 (35000 cellules/puits) ou CAL-1 transfectée de façon stable par un vecteur d'expression comprenant une molécule d'acide nucléique codant pour l'antigène CD303 humain sont incubées dans une plaque 96 puits à fond plat avec des cellules NK et des concentrations croissantes d'anticorps anti-CD303 pendant 4 heures à 37°C. Après incubation, le surnageant est collecté. La lyse des cellules cibles induite par les anticorps anti-CD303 est mesurée de façon chromogénique en quantifiant l'enzyme intracellulaires lactate déshydrogénase (LDH) relarguée dans le surnageant par les cellules cibles lysées (Roche Diagnostics - Cytotoxicity Détection Kit LDH).

Le pourcentage de lyse est calculé selon la formule suivante :

$$\% \text{ lyse} = [(ER - SR) / (100 - SR)] - [(NC - SR) / (100 - SR)]$$

Où ER et SR représentent respectivement les relargages expérimental (ER) et spontané (SR) de LDH, et NC représente la cytotoxicité naturelle des cellules NK.

Les résultats (% lyse) sont exprimés en fonction du facteur de dilution de l'anticorps. Pour chaque anticorps, la valeur d'« activité 50% » correspond au facteur de dilution de l'anticorps nécessaire pour induire 50% de la valeur plateau obtenue pour cet anticorps. Cette valeur peut être calculée avec le logiciel PRISM.

*Caractérisation de l'activité d'induction de sécrétion de cytokines*

Dans un autre mode de réalisation, l'utilisation des lignées cellulaires selon l'invention vise à caractériser l'activité d'induction de sécrétion de cytokines de l'anticorps anti-CD303 humain. Ainsi, l'invention concerne l'utilisation de la lignée cellulaire selon l'invention pour caractériser l'activité d'induction de sécrétion de cytokines d'un anticorps dirigé contre l'antigène CD303 humain.

Par « activité d'induction de sécrétion de cytokines », on entend la capacité de l'anticorps à induire la sécrétion de différentes cytokines par des cellules effectrices du système immunitaire.

La mesure de l'activité d'induction de sécrétion de cytokines d'un anticorps est réalisée par des techniques conventionnelles. Notamment, dans un mode de réalisation, l'invention concerne un procédé de test de l'activité d'induction de sécrétion de cytokines d'un anticorps dirigé contre l'antigène CD303 humain, comprenant les étapes suivantes :

a) mise en contact de la lignée cellulaire selon l'invention avec un anticorps dirigé contre l'antigène CD303 humain et des cellules effectrices ;
b) incubation pendant une durée appropriée pour permettre la sécrétion par les cellules effectrices d'au moins une cytokine; et
c) mesure du taux de sécrétion d'au moins une cytokine.

A l'étape a), la lignée cellulaire selon l'invention (cellules cibles) est mise en contact avec un anticorps dirigé contre l'antigène CD303 humain à caractériser (tel que décrit ci-dessus) et des cellules effectrices. Les cellules effectrices susceptibles d'être utilisées et les conditions de mise en contact sont les mêmes que celles susceptibles d'être utilisées pour la caractérisation de l'ADCC (voir ci-dessus).

A l'étape b), on réalise une incubation (généralement à 37°C) pendant une durée appropriée pour permettre la sécrétion par les cellules effectrices d'au moins une cytokine. La durée d'incubation peut varier entre quelques heures et environ une journée, notamment entre 1 et 24 heures, en particulier pendant 4 à 24 heures. L'homme du métier sait déterminer une durée optimale en fonction des conditions particulières du test.

Enfin, à l'étape c), on mesure le taux de sécrétion d'au moins une cytokine, par des technologies classiques bien connue de l'homme du métier, telles que le dosage par cytométrie de flux à partir de surnageants de culture. Les cytokines testées peuvent notamment inclure l'IL-2, l'IL-10, l'IFN-$\gamma$ et le TNF-$\alpha$.

*Caractérisation de l'activité CDC (activité via le complément)*

Dans un autre mode de réalisation, l'utilisation des lignées cellulaires selon l'invention vise à caractériser l'activité CDC de l'anticorps anti-CD303 humain. Ainsi, l'invention concerne l'utilisation de la lignée cellulaire selon l'invention pour caractériser l'activité CDC d'un anticorps dirigé contre l'antigène CD303 humain.

Par « activité CDC » (CDC étant l'abréviation de « complément dependent cytotoxicity », soit « cytotoxicité dépendante du complément »), on entend la capacité de l'anticorps à induire la mort de cellules cibles (ici, les cellules de la lignée cellulaire selon l'invention) en présence des protéines du complément, par sa liaison d'une part aux cellules cibles par l'intermédiaire de ses parties variables se liant à l'antigène situé à la surface des cellules cibles, et d'autre part aux protéines du complément.

La mesure de l'activité CDC d'un anticorps est réalisée par des techniques conventionnelles. Notamment, dans un mode de réalisation, l'invention concerne un procédé de test de l'activité CDC d'un anticorps dirigé contre l'antigène CD303 humain, comprenant les étapes suivantes :

a) mise en contact de la lignée cellulaire selon l'invention avec un anticorps dirigé contre l'antigène CD303 humain et d'une source de protéines du complément ;
b) incubation pendant une durée appropriée pour permettre la lyse des cellules de la lignée cellulaire selon l'invention de l'étape a); et
c) mesure du taux de lyse des cellules de la lignée cellulaire selon l'invention de l'étape a).

A l'étape a), la lignée cellulaire selon l'invention (cellules cibles) est mise en contact avec un anticorps dirigé contre l'antigène CD303 humain à caractériser (tel que décrit ci-dessus) et une source de protéines du complément, généralement du sérum.

Là encore, les cellules cibles (cellules qui expriment à leur surface l'antigène CD303 humain reconnu par l'anticorps à tester, ici les cellules de la lignée cellulaire selon l'invention) peuvent avoir préalablement été marquées de façon intracellulaire par un marqueur permettant à l'étape c) de mesurer le nombre de cellules cibles effectivement lysées. Tout marqueur approprié, tel qu'un marqueur radioactif, fluorescent, luminescent, ou chromogénique peut être utilisé. De manière alternative, les cellules cibles peuvent ne pas être marquées préalablement. On peut alors utiliser le dosage d'une protéine présente dans les cellules cibles (telle que l'enzyme LDH présente dans tous types de cellules) pour mesurer le nombre de cellules lysées à l'étape c).

Là encore, on réalise généralement une courbe dose/réponse d'ADCC. Pour cela, des quantités croissantes d'anticorps sont mises en contact avec les cellules cibles et les cellules effectrices dans des puits séparés et l'ADCC mesurée à différentes concentrations d'anticorps. Chaque point est généralement réalisé en plusieurs exemplaires (duplicats ou triplicats notamment).

Là encore, on réalise généralement des contrôles permettant d'estimer à l'étape c) le pourcentage de lyse obtenu à chaque concentration d'anticorps testée. Pour cela, on réaliser une gamme de calibration obtenue avec différentes dilutions de cellules cibles lysées par un produit chimique tel qu'un détergent (Triton X-100 2% par exemple) correspondant à respectivement à différents pourcentages de lyse (par exemple 100, 50, 25 et 0% de lyse). Les contrôles incluent également le relargage spontané (cellules cibles seules).

A l'étape b), on réalise une incubation (généralement à 37°C) pendant une durée appropriée pour permettre la lyse des cellules cibles (cellules de la lignée cellulaire selon l'invention). La durée d'incubation peut varier entre quelques heures et environ une journée, notamment entre 1 et 24 heures, en particulier pendant 1 à 2 heures. L'homme du métier sait déterminer une durée optimale en fonction des conditions particulières du test.

Enfin, à l'étape c), on mesure le taux de lyse des cellules cibles (cellules de la lignée cellulaire selon l'invention). Les résultats sont calculés selon la formule suivante :

% de lyse = (% de lyse avec anticorps et complément) – (% de lyse avec anticorps sans complément).

Un exemple précis de test CDC selon l'invention est le suivant :
Des cellules Jurkat chaine Fc Gamma-CD303 ou CAL-1 transfectée de façon stable par un vecteur d'expression comprenant une molécule d'acide nucléique codant pour l'antigène CD303 humain sont incubées avec des concentrations croissantes d'anticorps anti-CD303 (0 to 5000 ng/ml) et en présence de sérum de jeunes lapins comme source de (dilution au 1/10).

Après 2h d'incubation à 37°C, la quantité d'enzyme intracellulaires lactate déshydrogénase (LDH) relarguée dans le surnageant par les cellules cibles lysées est mesurée avec le kit « Cytotoxicity Detection LDH » (Roche Diagnostics réf 11644793001).

*Caractérisation de l'apoptose induite par l'anticorps*

Dans un autre mode de réalisation, l'utilisation des lignées cellulaires selon l'invention vise à caractériser l'activité d'apoptose de l'anticorps anti-CD303 humain. Ainsi, l'invention concerne l'utilisation de la lignée cellulaire selon l'invention pour caractériser l'activité d'induction d'apoptose d'un anticorps dirigé contre l'antigène CD303 humain.

Par « activité d'apoptose », on entend la capacité de l'anticorps à caractériser à induire l'apoptose de cellules exprimant l'antigène reconnu par l'anticorps.

La mesure de l'activité d'apoptose d'un anticorps est réalisée par des techniques conventionnelles, telles que des tests d'apoptose classiques en cytométrie de flux utilisant l'iodure de propidium et l'Annexine V. Notamment, dans un mode de réalisation, l'invention concerne un procédé de test de l'activité d'apoptose d'un anticorps dirigé contre l'antigène CD303 humain, comprenant les étapes suivantes :

a) mise en contact de la lignée cellulaire selon l'invention avec un anticorps dirigé contre l'antigène CD303 humain;
b) incubation pendant une durée appropriée pour permettre l'induction par l'anticorps de l'apoptose des cellules de la lignée cellulaire selon l'invention de l'étape a); et

c) mesure du taux de cellules apoptotiques de la lignée cellulaire selon l'invention de l'étape a).

A l'étape a), la lignée cellulaire selon l'invention (cellules cibles) est mise en contact avec un anticorps dirigé contre l'antigène CD303 humain à caractériser (tel que décrit ci-dessus), en présence ou en absence d'un crosslinker (anticorps ou fragment d'anticorps dirigé contre le fragment Fc par exemple).

A l'étape b), on réalise une incubation (généralement à 37°C) pendant une durée appropriée pour permettre la lyse des cellules cibles (cellules de la lignée cellulaire selon l'invention). La durée d'incubation peut varier entre quelques heures et environ une journée, notamment entre 1 et 24 heures, en particulier pendant 16 heures. L'homme du métier sait déterminer une durée optimale en fonction des conditions particulières du test.

Enfin, à l'étape c), on mesure le taux de cellules apoptotiques. Cette mesure peut être réalisée en cytométrie de flux, en utilisant un marquage des cellules à l'iodure de propidium (PI) et à l'Annexine V. La combinaison de ces deux marqueurs permet de différencier les cellules vivantes (Annexine-V et IP négatives) des cellules en phase précoce d'apoptose (Annexine-V positives, IP négatives) ou en phase tardive d'apoptose (Annexine-V positives, IP positives).

Un exemple précis de test de l'activité d'apoptose selon l'invention est le suivant : Les cellules cibles (cellules d'une lignée cellulaire selon l'invention, $2,5 \times 10^5$) sont incubées avec l'anticorps anti-CD303 à caractériser ($1 \mu g/ml$) avec ou sans crosslinker (par exemple F(ab2)' de chèvre anti-Fc$\gamma$ d'IgG humaines à 10 $\mu g/ml$) dans 1 ml de RMPI 10% SVF, dans des plaques P24 pendant 24h à 37°C. Les cellules sont ensuite centrifugées, lavées deux fois en PBS, reprises dans le tampon fourni par le kit et incubées avec l'Annexine V-FITC et l'iodure de propidium (IP) selon les recommandations de BD Biosciences. Les cellules sont analysées au cytomètre en flux, le pourcentage de cellules apoptotiques correspond aux cellules marquées par l'annexine V (annexine V et annexine V +IP).

*Caractérisation de l'activité phagocytose*

Dans un autre mode de réalisation, l'utilisation des lignées cellulaires selon l'invention vise à caractériser l'activité de phagocytose de l'anticorps anti-CD303 humain. Ainsi, l'invention concerne l'utilisation de la lignée cellulaire selon l'invention pour caractériser l'activité d'induction de phagocytose d'un anticorps dirigé contre l'antigène CD303 humain.

Par « activité de phagocytose », on entend la capacité de l'anticorps à induire la phagocytose de cellules cibles exprimant l'antigène reconnu par l'anticorps par des cellules effectrices du système immunitaire capables de phagocyter (monocytes, macrophages, neutrophiles et cellules dendritiques), par sa liaison d'une part aux cellules cibles par l'intermédiaire de ses parties variables se liant à l'antigène situé à la surface des cellules cibles, et d'autre part aux cellules effectrices par l'intermédiaire de la liaison de son fragment Fc à un récepteur Fc (FcR ; Fc$\gamma$RI, Fc$\gamma$RIIA, Fc$\gamma$RIIB2 dans le cas des IgG) situé à la surface des cellules effectrices.

La mesure de l'activité ADCC d'un anticorps est réalisée par des techniques conventionnelles. Notamment, dans un mode de réalisation, l'invention concerne un procédé de test de l'activité ADCC d'un anticorps dirigé contre l'antigène CD303 humain, comprenant les étapes suivantes :

a) mise en contact de la lignée cellulaire selon l'invention avec un anticorps dirigé contre l'antigène CD303 humain et des cellules effectrices capables de phagocyter ;

b) incubation pendant une durée appropriée pour permettre la phagocytose des cellules de la lignée cellulaire selon l'invention de l'étape a); et

c) mesure du taux de phagocytose des cellules de la lignée cellulaire selon l'invention de l'étape a).

A l'étape a), la lignée cellulaire selon l'invention (cellules cibles) est mise en contact avec un anticorps dirigé contre l'antigène CD303 humain à caractériser (tel que décrit ci-dessus) et des cellules effectrices capables de phagocyter. Dans le contexte de la caractérisation de l'activité de phagocytose, les cellules effectrices doivent être capables de phagocyter. Il peut s'agir de monocytes, macrophages, ou neutrophiles. Dans le cas des IgG, la phagocytose est réalisée via l'un des récepteurs Fc$\gamma$RI, Fc$\gamma$RIIA, et Fc$\gamma$RIIB2 présent à la surface des cellules effectrices capables de phagocyter. Le récepteur Fc$\gamma$RI étant de forte affinité, contrairement aux récepteurs Fc$\gamma$RIIA et Fc$\gamma$RIIB2, on peut mesurer l'activité de phagocytose via Fc$\gamma$RIIA et Fc$\gamma$RIIB2 en utilisatn des IgG polyvalentes telles que décrites ci-dessus pour l'ADCC.

Les cellules cibles (cellules qui expriment à leur surface l'antigène CD303 humain reconnu par l'anticorps à tester, ici les cellules de la lignée cellulaire selon l'invention) peuvent avoir préalablement été marquées de façon intracellulaire par un marqueur permettant à l'étape c) de mesurer le nombre de cellules cibles effectivement phagocytées. Tout marqueur approprié, tel qu'un marqueur radioactif, fluorescent, luminescent, ou chromogénique peut être utilisé.

Les cellules cibles (cellules qui expriment à leur surface l'antigène CD303 humain reconnu par l'anticorps à tester, ici les cellules de la lignée cellulaire selon l'invention) et les cellules effectrices sont présentes dans un ratio effecteur/cible (E/C) proche des conditions physiologiques *in vivo*.

On réalise généralement une courbe dose/réponse d'activité de phagocytose. Pour cela, des quantités croissantes d'anticorps sont mises en contact avec les cellules cibles et les cellules effectrices dans des puits séparés et l'activité de phagocytose mesurée à différentes concentrations d'anticorps. Chaque point est généralement réalisé en plusieurs exemplaires (duplicats ou triplicats notamment).

Un contrôle positif est également réalisé: phagocytose naturelle (NP), obtenue en mettant en contact les cellules cibles uniquement avec les cellules effectrices (sans anticorps).

A l'étape b), on réalise une incubation (généralement à 37°C) pendant une durée appropriée pour permettre la phagocytose des cellules cibles (cellules de la lignée cellulaire selon l'invention). La durée d'incubation peut varier entre quelques heures et environ une journée, notamment entre 1 et 24 heures, en particulier pendant 4 à 24 heures. L'homme du métier sait déterminer une durée optimale en fonction des conditions particulières du test.

Enfin, à l'étape c), on mesure le taux de phagocytose des cellules cibles (cellules de la lignée cellulaire selon l'invention). Ceci est généralement réalisé en éliminant les cellules cibles non phagocytées par lavages (les cellules effectrices capables de phagocyter étant généralement adhérentes) et en mesurant la quantité de cellules cibles préalablement marquées présente dans les phagocytes.

Le pourcentage de phagocytose ou index de phagocytose peut alors être calculé selon la formule suivante :

% phagocytose = (% de cellules effectrices contenant au moins une cellule cible) x (nombre moyen de cellules cibles par cellule effectrice contenant des cellules cibles). Les résultats (%phagocytose) sont exprimés en fonction du facteur de dilution de l'anticorps. Pour chaque anticorps, une valeur d'« activité 50% » ou « EC50 »peut être calculée, qui correspond au facteur de dilution de l'anticorps nécessaire pour induire 50% de la valeur plateau obtenue pour cet anticorps.

*Comparaison de l'affinité de plusieurs anticorps anti-CD303 pour l'antigène CD303 humain*

Dans un autre mode de réalisation, l'invention concerne l'utilisation de la lignée cellulaire selon l'invention pour comparer l'affinité de liaison de plusieurs anticorps anti-CD303 à l'antigène CD303 humain.

L'affinité de liaison d'un anticorps anti-CD303 à l'antigène CD303 humain peut être mesurée en utilisant une lignée cellulaire selon l'invention par toute technologie connue de l'homme du métier.

Notamment, la fixation des anticorps anti-CD303 à tester sur le CD303 humain exprimé par la lignée cellulaire selon l'invention peut être étudiée par cytométrie en flux. Pour cela, les cellules de la lignée cellulaire selon l'invention sont dans un premier temps incubées pendant 30 minutes à 4°C avec un anticorps (anti-CD303 ou contrôle négatif) à différentes concentrations (0-40 $\mu$g/mL, concentration finale). Après lavage avec le diluant, les anticorps sont visualisés par ajout d'un fragment F(ab')$_2$ d'IgG de chèvre anti-souris couplé à la phycoérythrine (PE) (100 $\mu$L à une dilution de 1:100 dans le diluant) pendant 45 minutes à 4°C. Les cellules sont ensuite lavées et analysées par cytométrie en flux (FC500, Beckman Coulter).

Les résultats sont exprimés sous forme de courbe dose-réponse, les concentrations étant reportées sur l'axe des X et les moyennes d'intensité de fluorescence (MFI) sur l'axe des Y.

Les valeurs au plateau (Bmax, moyenne d'intensité de fluorescence maximum) peuvent être estimées après modélisation des courbes, par exemple par le logiciel GraphPad PRISM® et comparées entre les différents anticorps anti-CD303 à tester. On peut également calculée une valeur dénotée EC$_{50}$, représentant la quantité d'anticorps requise pour atteindre 50% de la valeur Bmax. Les valeurs d'EC$_{50}$ des différents anticorps à tester peuvent être comparées.

*Comparaison des épitopes de l'antigène CD303 humain reconnus par deux anticorps anti-CD303*

Dans un autre mode de réalisation, l'invention concerne l'utilisation de la lignée cellulaire selon l'invention pour comparer les épitopes de l'antigène CD303 humain reconnus par plusieurs anticorps anti-CD303.

Afin de comparer les épitopes de l'antigène CD303 humain reconnus par deux anticorps anti-CD303, les anticorps à tester peuvent être mis en compétition pour la liaison à une lignée cellulaire selon l'invention avec différents anticorps anti-CD303 dont l'épitope est connu. Notamment, l'un des deux anticorps à tester est de préférence porteur d'un marqueur de détection, et est mis en contact avec une lignée cellulaire selon l'invention en absence ou en présence de concentrations croissantes de l'autre anticorps anti-CD303 non marqué. L'intensité de marquage des cellules de la lignée cellulaire selon l'invention est alors mesurée, Si les épitopes reconnus par les deux anticorps sont identiques ou se chevauchent, une compétition entre les deux anticorps pour la fixation à la lignée cellulaire selon l'invention est observée, c'est à dire que l'intensité de marquage des cellules de la lignée cellulaire selon l'invention diminue lorsque la concentration d'anticorps non marqué augmente.

Si l'épitope de l'antigène CD303 humain reconnu par l'un des deux anticorps anti-CD303 est connu, cela peut permettre de caractériser l'épitope reconnu par l'autre anticorps anti-CD303.

Caractérisation des capacités fonctionnelles des cellules effectrices d'un sujet humain

La lignée cellulaire selon l'invention peut également être utilisée pour caractériser les capacités fonctionnelles (notamment les capacités de type ADCC, CDC, sécrétion de cytokines, apoptose, et phagocytose) des cellules effectrices d'un sujet humain, en particulier un sujet humain souffrant de BPDCN ou d'une maladie autoimmune.

*Capacités fonctionnelles à caractériser*

Les capacités fonctionnelles susceptibles d'être caractérisées sont les mêmes que précédemment, notamment les capacités de type ADCC, CDC, sécrétion de cytokines, apoptose, et phagocytose.
Les méthodes de test sont les mêmes que décrit précédemment pour la caractérisation d'anticorps anti-CD303, à l'exception du fait qu'un même anticorps est utilisé et que les contrôles utilisés sont des contrôles de cellules effectrices dont les capacités fonctionnelles sont connues.

*Cellules effectrices à caractériser*

Les cellules effectrices à caractériser peuvent notamment avoir été préalablement obtenues de sujets humains souffrant de BPDCN ou d'une maladie autoimmune.
Les cellules effectrices du sang périphérique peuvent être utilisées pour caractérisation, sous forme de PBMC, ou de cellules effectrices purifiées, telles que des monocytes, macrophages, lymphocytes T, ou cellules NK.

*Kits de caractérisation d'anticorps anti-CD303*

Selon un autre objet, l'invention consiste en un kit de caractérisation d'anticorps anti-CD303 comprenant la lignée cellulaire selon l'invention, et avantageusement au moins un réactif choisi parmi des cellules effectrices, des IgG polyvalentes, et des anticorps anti-CD303 de référence.

**EXEMPLES**

***Exemple 1. Préparation et caractérisation d'une lignée cellulaire Jurkat exprimant de façon stable le CD303 humain et la chaine gamma du récepteur FcεRI humain***

Matériels et méthodes

*Cellules et conditions de culture*

La lignée cellulaire Jurkat Fc chaine γ (lignée Jurkat Clone E6-1 (ATCC® TIB-152™) transfectée de façon stable par un vecteur d'expression (pcDNA4/TO) de la chaine gamma du récepteur FcεRI humain) a été cultivée dans du milieu RPMI 1640 avec de la glutamine supplémenté avec 10% de sérum de veau fœtal (SVF) à 37°C sous 7% de $CO_2$ dans l'air. Les cellules ont été ensemencées à $0,5 \times 10^5$ cellules/ml trois jours avant Nucleofection®.

*Construction du vecteur*

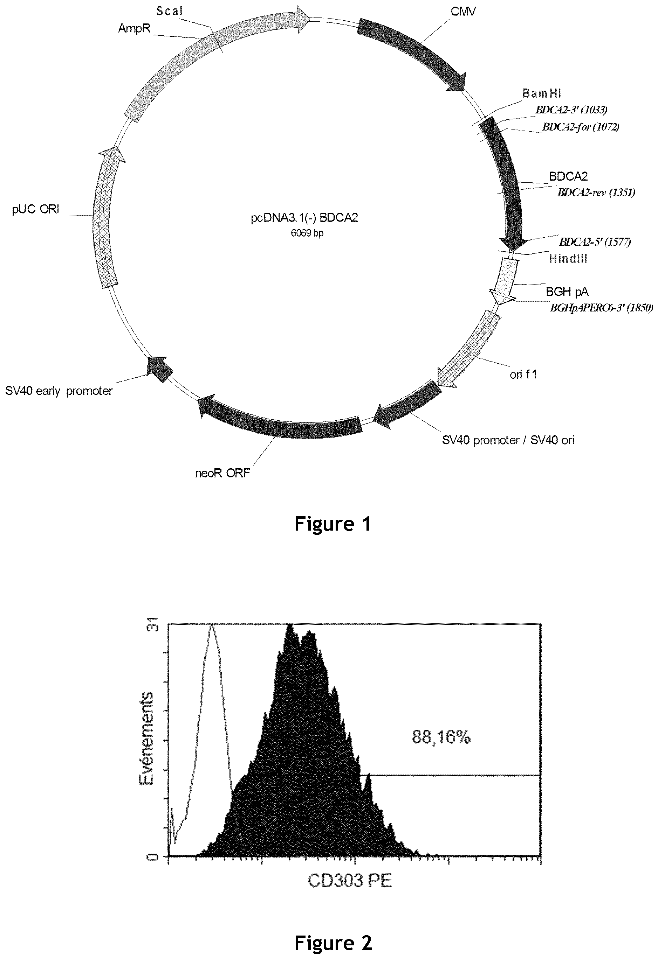

L'ADNc pleine longueur codant pour l'antigène CD303 humain (GenBank / EMBL / DDBL, AF293615.1) a été sous-cloné dans le vecteur pcDNA3.1 (-) (voir carte de ce vecteur sur la **Figure 1**).
Après ajout des sites de restriction BamHI et HindIII, de la séquence de kozak et d'un deuxième codon STOP, la séquence codante est synthétisée chez MWG.
Le vecteur pcDNA3.1 a été séquencé chez MWG avec l'amorce BGHpAPERC6-3' avant de démarrer l'étude afin de déterminer si le vecteur est (+) ou (-) car les sites de clonages sont différents dans ces deux vecteurs. Le séquençage a montré que le vecteur est le pcDNA3.1(-).
La séquence codante de CD303 et les amorces CD303-5' et CD303-3' sont synthétisé chez MWG. Une double digestion BamHI et Hind III est réalisée sur le vecteur MWG comportant la séquence codante de CD303et du vecteur pcDNA3.1(-).La digestion est vérifier sur gel et les séquences sont purifiées par Nucleospin extract II. CD303est inséré dans le vecteur pcDNA3.1(-) par ligation, puis transformé dans *E. Coli.* Criblage par PCR de 7 colonies bactériennes en utilisant l'amorce sens CD303-5' : 5'-ACATTCACTGTCATGTACCTCAGAAGTC-3' ; et l'amorce antisens BGHpAPERC6-3' : 5'-CATGCCTGCTATTGTCTTCCCA-3'. Le programme PCR utilisé comprend 30 cycles comprenant notamment les étapes d'hybridation (52°C), d'élongation (30 secondes à 72°C) et de dénaturation (95°C). 6 colonies sur 7 contiennent l'insert CD303 (produit PCR de 295 pb). A partir de ces 6 colonies 4 glycérols stock sont préparée, et 4 cultures bactériennes sont purifiées afin de récupérer le vecteur pcDNA3.1(-) portant l'insert CD303. Une digestion de contrôle avec les enzymes BamHI et HindIII est réalisée afin de s'assurer de la présence de l'insert CD303.

La séquence des inserts CD303présents sur les vecteurs pcDNA3.1(-) purifié par miniprep, est confirmer par séquançage grâce aux amorces CD303-5' et CD303-3' (5'-AGACCTTCAACTGGAACCACCAGAG-3').

Les vecteurs sont ensuite purifiés par Maxiprep Endofree et validés par séquençage avec les amorces CD303-5' et CD303-3'.

Les vecteurs sont ensuite linéarisé par ScaI et précipité avant d'être repris dans du tampon TE à 1$\mu$g/ml.

*Transfection des cellules Jurkat Fc chaine $\gamma$*

Les transfections ont été effectuées suivant les procédures établies des systèmes de transfection Amaxa (Amaxa Biosystems, Cologne, Allemagne). Pour la nucléofection, $2 \times 10^6$ cellules et 3 $\mu$g de vecteur pcDNA3.1 linéarisé (-) CD303 ont été remis en suspension dans 100 $\mu$L de solution Nucleofector (Kits Nucleofector ™ pour Jurkat, Amaxa) selon le programme X-001. Comme témoin, 1 $\mu$g d'ADN plasmidique EGFP (Enhanced Green Fluorescent Protein) (pmaxGFP, Amaxa) a été utilisé avec le même protocole de nucléofection.

Après la transfection, les cellules ont été étalées dans des plaques à 6 puits sans antibiotique avec du milieu de culture. Le milieu sélectif a été en partie renouvelé (environ un demi-volume) deux fois par semaine avec un intervalle de 3-4 jours. Après 24h, les cellules transfectées avec pmaxGFP ont été recueillies pour mesures. Les efficacités de transfection ont été déterminées par FACS après transfection de pmax-GFP (Lonza, Allendale, NJ, USA).

Après 48h de culture, les cellules transfectées ont été analysées en cytométrie de flux et les cellules exprimant CD303 à leur surface ont été purifiées par tri cellulaire par FACS.

*Tri cellulaire activé par fluorescence*

Brièvement, l'ensemble des cellules transfectées ont été marquées avec des anticorps anti-CD303 marqués PE (Miltenyi Biotec). Des anticorps monoclonaux de souris de même isotype PE (phycoérythrine) ont servi de témoins. Les cellules marquées par fluorescence ont été analysées sur un trieur FACS EPICS ALTRA cellulaire (Beckman). La sélection par généticine a été démarrée 24 heures après le tri cellulaire, par application de 1 mg / ml de G418 (Sigma) dans du milieu de culture.

*Clonage par dilution limite*

Les cellules ont ensuite ensemencées à $1.10^5$ cellules / ml pendant 48 heures avant le clonage. Le jour du clonage, les cellules ont été comptées, la viabilité doit être supérieure à 80% pour continuer. Les cellules ont été remises en suspension dans du RPMI + 10% de SVF (2 cellules / ml) et ensemencées dans des plaques à 96 puits (200 $\mu$l / puits correspondant à 0,4 cellule/puits). Les cellules ont été incubées à 37°C, 7% CO2 pendant 10 à 12 jours.

Le milieu a été renouvelé en partie (environ un demi-volume) deux fois par semaine avec un intervalle de 3-4 jours.

*Détermination du nombre de molécules CD303 par cellule*

La détermination du nombre de molécules CD303 par cellule d'un des clones Jurkat chaine Fc gamma-CD303 est réalisée par cytométrie en flux, à l'aide du kit Qifikit commercialisé par Dako, et de l'anticorps murin anti-CD303 AC144.

Des cellules d'un des clones Jurkat chaine Fc Gamma-CD303 et les anticorps sont préparés dans du diluant (PBS + 1% SVF).

1 x $10^5$ cellules sont incubées à 4°C pendant 30 minutes avec 100 $\mu$L d'anticorps (anti-CD303 ou contrôle négatif) à différentes concentrations (0-40 $\mu$g/mL, concentration finale).

Après lavage avec le diluant, les anticorps sont visualisés par ajout d'un fragment F(ab')$_2$ d'IgG de chèvre anti-souris couplé à la phycoérythrine (PE) (100 $\mu$L à une dilution de 1:100 dans le diluant) pendant 45 minutes à 4°C. Les cellules sont ensuite lavées et analysées par cytométrie en flux (FC500, Beckman Coulter).

Résultats

*Sélection par FACS de cellules Jurkat Fc chaine $\gamma$ transfectées par CD303*

Avant le tri cellulaire, près de 0,4% des cellules exprimaient CD303 (données non montrées).

Les cellules CD303 positives ont été isolées par FACS (20 767 cellules, ensemble 008-2) et ensemencées dans du milieu RPMI + 10% de SVF. Malheureusement, ces cellules ne poussent pas dans ces conditions de culture.

Les cellules CD303 négatives ont été ensemencées sous sélection généticine (Jour 3). Au jour 25, 35% de ces cellules exprimaient CD303. Après un deuxième tri cellulaire, presque 73% des cellules étaient positives pour CD303 et après encore 18 jours sous sélection à la généticine, plus de 88% de cellules exprimaient CD303 (**Figure 2**).

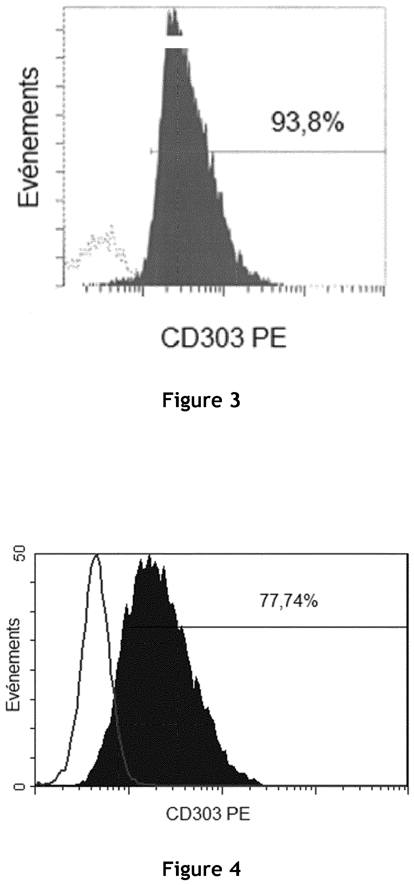

Au jour 44, après un troisième tri cellulaire, presque 93% des cellules exprimaient CD303 (**Figure 3**).

Stabilité de l'expression de CD303 : après deux mois de la culture sous sélection généticine, plus de 77% des cellules exprimaient encore CD303 (**Figure 4**).

*Clonage par dilution limite de cellules CD303 +*

En outre, nous avons effectué un clonage par dilution limite des cellules Jurkat Fc chaine γ transfectants stables exprimant CD303.

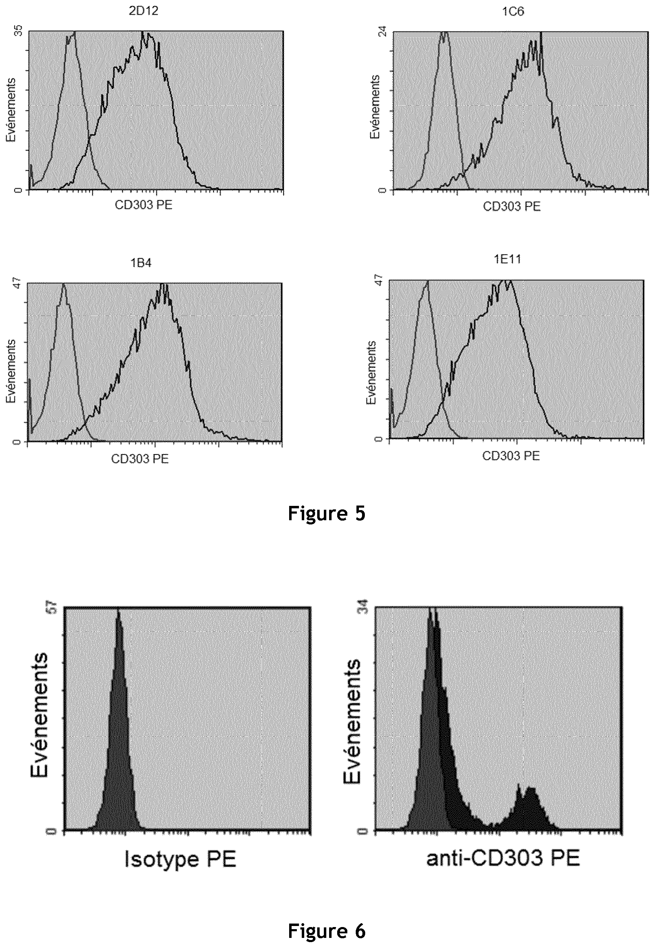

27 clones ont été isolés et testés par coloration avec un anticorps anti- CD303-PE. Seuls quatre clones ont été sélectionnés et décongelés, plus de 80% des cellules exprimaient CD303 (**Figure 5**).

*Détermination du nombre de molécules CD303 par cellule*

L'un des clones a ensuite été caractérisé concernant le nombre de molécules CD303 par cellule. Les résultats montrent un nombre de molécules CD303 par cellule de 28325 +/- 5405.

Conclusion

Après la transfection avec un vecteur pcDNA3.1 (-) CD303, la sélection de généticine et le tri cellulaire activé par fluorescence, un pool stable de cellules Jurkat Fc chaine γ CD303+ (plus de 80% de cellules positives) est maintenant disponible et représente un outil intéressant pour la caractérisation in vitro d'anticorps anti- CD303.

**Exemple 2. *Transfection de cellules pDC pour surexpression de CD303***

La transfection des cellules CAL-1 (pDC) pour surexprimer CD303 est réalisée avec le kit Amaxa Human Dendritic Cell Nucleofector.

La veille de la transfection les cellules sont repiquées à $2.10^5$ cellules/ml. Le jour de la transfection les cellules CAL-1 ont une densité cellulaire de 4,6 $10^5$ cellules/ml et une viabilité à 85%.

Le jour de la transfection :

- le milieu EMS+10% SVF (sérum de veau foetal) est préchauffé durant 1 heure à 37°C (1 flasque F25 contenant 7.5 ml et 2 puits de plaque P6 contenant 1.5ml),
- le « supplément » disponible dans le kit (135μl) est ajouté à la solution « Nucleofector » (615μl).
- $7.10^6$ cellules sont centrifugées 10 minutes à 200g ($1.10^6$ cellules/cuvette).
- Le culot est alors repris dans 700μl de solution Nucleofector et réparti à 100μl par eppendorf.
- 3 μl (3μg) de vecteur pcDNA3.1- CD303, 4μl (2μg) de vecteur pmax-GFP ou pas de vecteur (témoin négatif) sont ajoutés par eppendorf.
- La suspension cellulaire est transférée dans les cuvettes et le programme U-002 est appliqué.
- 500μl de milieu préchauffé sont ajoutés dans la cuvette et la suspension cellulaire est transférée délicatement dans la F25 ou les P6 contenant du milieu :

  - 5 cuvettes comprenant pcDNA3.1- CD303sont reprises en F25 dans 10 ml de milieu ;
  - 1 cuvette comprenant Pmax-GFP est reprise en P6 dans 2 ml de milieu ;
  - 1 cuvette comprenant T- est reprise en P6 dans 2 ml de milieu.

- Puis les cuvettes sont laissées à incuber à 37°C avec 7% de CO2.

**De J+1 à J+51:**

- **A J+1 :** Des mesures de GFP par cytométrie et par microscopie à fluorescence sont réalisées pour estimer l'efficacité de transfection. Les mesures de cytométrie révèlent 49% de cellules transfectées et celles de microscopie 43% de cellules transfectées.
- **A J+3 :** Comptage des cellules et reprise du culot à $5.10^5$ cellules/ml en milieu de sélection (EMS + 10% SVF + 1 g/L G418).

- **A J+6 :** Comptage des cellules et reprise du culot à 4.10$^5$ cellules/ml en milieu de sélection (EMS + 10% SVF + 1 g/L G418).

  ◦ Cal-1 transfectée CD303: 8,1.10$^5$ cellules /ml et viabilité à 49%

  ◦ Cal-1 témoin négatif : 2,6.10$^5$ cellules/ml et viabilité à 42.6%

- **A J+8 :** Comptage des cellules et mise en P24 d'une partie des cellules (1ml/puits) et le reste en F150 (56ml)

  ◦ Cal-1 transfectée CD303: 0,22.10$^5$ cellules /ml et viabilité à 3%

  ◦ Cal-1 témoin négatif : 0,1.10$^5$ cellules/ml et viabilité à 1,5%

- **A J+10 :** Comptage des cellules Cal-1 transfectée CD303

  ◦ F150 : 0,08.10$^5$ cellules /ml et viabilité à 1,3%. Centrifugation et reprise du culot dans 65 ml pour ne pas dépasser 5.10$^5$ cellules mortes/ml

  ◦ P24 : Changement de la moitié du milieu

  ◦ Témoin négatif : 0,0.10$^5$ cellules /ml et viabilité à 0% → Arrêt.

- **A J+13 :** Comptage des cellules Cal-1 transfectée CD303

  ◦ F150 : 0,04.10$^5$ cellules /ml et viabilité à 0,7%. Centrifugation et reprise du culot dans 70 ml pour ne pas dépasser 5.10$^5$ cellules mortes /ml

  ◦ P24 : Changement de la moitié du milieu

- **A J+17 :** Comptage des cellules Cal-1 transfectée CD303

  ◦ F150 : 0,04.10$^5$ cellules /ml et viabilité à 1,0%. Centrifugation et reprise du culot dans 56 ml pour ne pas dépasser 5.10$^5$ cellules mortes /ml

  ◦ P24 : Changement de la moitié du milieu

- **A J+20 :** Comptage des cellules Cal-1 transfectée CD303

  ◦ F150 : 0,002.10$^5$ cellules /ml et viabilité à 0,5%. → Arrêt.

  ◦ P24 : comptage d'un puits : 16,4.10$^5$ cell/ml et viabilité à 53,8% P24 transférée dans 3 F75 (8 puits de P24/F75 dans 30 ml final).

- **A J+22 :** Comptage des cellules Cal-1 transfectée CD303

  ◦ F75: 12,8.10$^5$ cellules /ml et viabilité à 85%.

  ◦ Marquage du pool avec l'anti-CD303-PE. Seule une petite proportion de la population cellulaire exprime CD303 à un fort niveau (voir **Figure 6**).

  ◦ Congélation de 8 cryotubes en azote liquide.

  ◦ Repiquage à 3.10$^5$ cellules/ml en F75

- **A J+24 :** Comptage des cellules Cal-1 transfectée CD303

  ◦ F75 : 19,9.10$^5$ cellules /ml et viabilité à 95%. Repiquage à 2.10$^5$ cellules/ml en F75

○ Clonage à 40 cellules/P96 en EMS + 10% SVF + 0.5g/l G418 et réalisation de 5 plaques de clonage.

• **De J+28 à J+37 :** Comptage des cellules Cal-1 transfectée CD303

○ F75 : Repiquage à $2.10^5$ cellules/ml en F75 3x/semaine

○ Clonage : changement de la moitié du milieu 1x/semaine.

• **De J+38 à J+50 :** Comptage des cellules Cal-1 transfectée CD303

○ F75 : Repiquage à $2.10^5$ cellules/ml en F75 3x/semaine

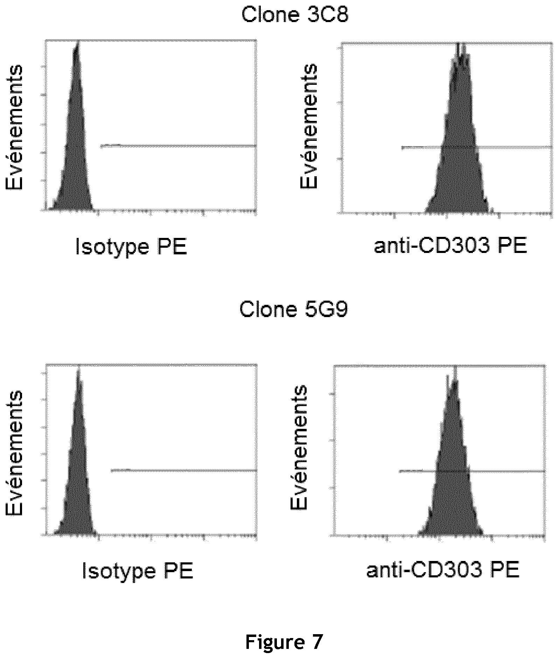

○ Clonage : Passage des clones en P24 puis P6 pour screening de l'expression de CD303 par cytométrie. Screening de 30 clones et conservation de 2 clones positifs, le NF-3C8 et le NF-5G9 (voir **Figure 7**).

• **A J+51 :** Congélation de 8 cryotubes des clones NF-3C8 et NF-5G9 et maintien des clones en culture à $0.5\ 10^5$ cellules/ml, repiquage 2x/semaine.

Les cellules de la lignée CAL-1 et les deux clones NF-3C8 et NF-5G9 ont ensuite été caractérisés quant au nombre de molécules CD303 par cellule, par cytométrie en flux, à l'aide du kit Qifikit commercialisé par Dako, et de l'anticorps murin anti-CD303 AC144, selon le protocole décrit à l'Exemple 1.

Les résultats montrent un nombre de molécules CD303 par cellule de 3000-6000 (soit environ 4500 en moyenne) pour les cellules de la lignée CAL-1 et de 40000 à 50000 (soit environ 45000 en moyenne) molécules CD303 par cellule pour les deux clones. Les deux clones NF-3C8 et NF-5G9 ont donc une forte expression de surface de CD303 par rapport à la lignée cellulaire d'origine CAL-1, avec un facteur d'amplification d'environ 10 (soit une augmentation d'environ 900% par rapport à la lignée cellulaire d'origine CAL-1).

### Exemple 3. Etude de stabilité des clones NF-3C8 et NF-5G9 en présence et en absence de G418 durant 3 mois.

Matériels et Méthodes

Les cellules sont repiquées 2x/semaine à $0.5\ 10^5$ cellules/ml en F25 (10ml) en milieu EMS + 10% SVF +/- G418 0.5g/L.

L'expression de CD303 est suivie toutes les 2 semaines par cytométrie.

Le marquage est réalisé sur glace en utilisant l'anti-CD303-PE (AC144) (Miltenyi biotec réf 130-090-511) dilué au $1/10^{ème}$ en PBS + 1%SVF.

Résultats

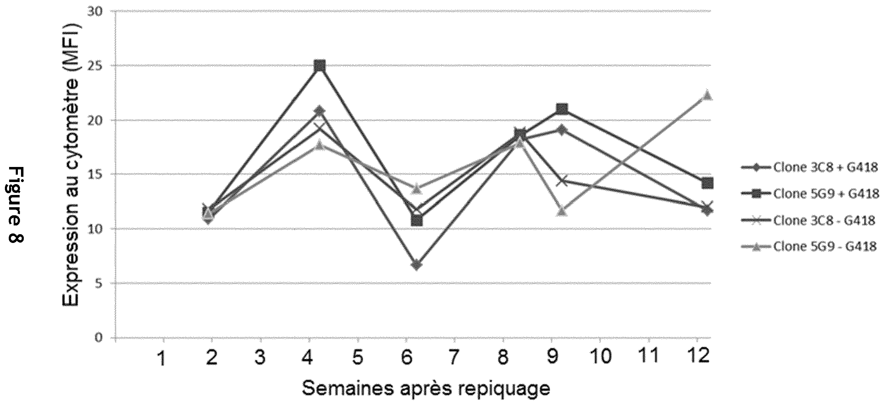

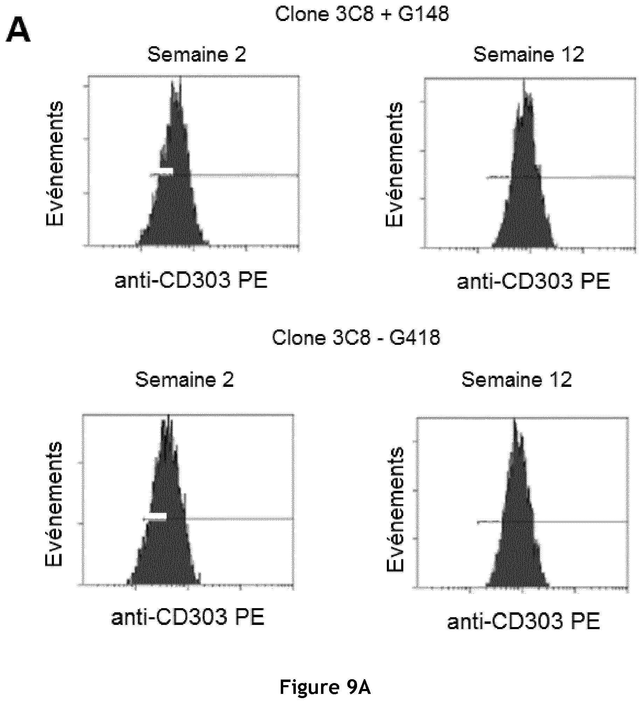

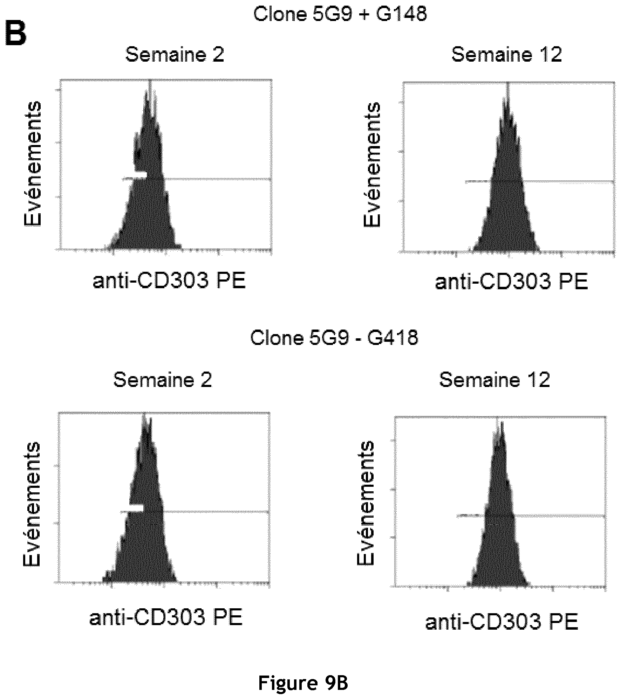

Les résultats des marquages en cytométrie sont présentés sur les **Figures 8 et 9.**

Les valeurs de MFI sont légèrement variables selon les tests mais on n'observe pas de perte d'expression de CD303 après 12 semaines de culture en absence de G418. L'expression de CD303 est donc stable pour les 2 clones testés.

### Exemple 4. Caractérisation d'anticorps chimériques anti-CD303 à l'aide des cellules Jurkat-CD303 ou des clones NF-3C8 et NF-5G9

Matériels et méthodes

*Anticorps*

Cinq anticorps chimériques anti-(CD303 humain) ont été testés, nommés 122A2, 102E9, 114D11, 104C12, et 104E10. Un anticorps contrôle dirigé contre un autre antigène et un anticorps humanisé anti-(CD303 humain) dénommé BIIB059 ont également été utilisés.

*Test de liaison à CD303*

Des cellules Jurkat chaine Fc Gamma-CD303 ou des cellules NF-3C8 et NF-5G9 d'une part et les anticorps, d'autre part, sont préparés dans du diluant (PBS + 1% SVF).

1 x $10^5$ cellules sont incubées à 4°C pendant 30 minutes avec 100 μL d'anticorps (anti-CD303 ou contrôle négatif) à différentes concentrations (0-40 μg/mL, concentration finale).

Après lavage avec le diluant, les anticorps sont visualisés par ajout d'un fragment F(ab')$_2$ d'IgG de chèvre anti-souris couplé à la phycoérythrine (PE) (100 μL à une dilution de 1:100 dans le diluant) pendant 45 minutes à 4°C. Les cellules sont ensuite lavées et analysées par cytométrie en flux (FC500, Beckman Coulter).

*Test ADCC via CD16a*

Des cellules Jurkat chaine Fc Gamma-CD303 (35000 cellules/puits) sont incubées dans une plaque 96 puits à fond plat avec des cellules NK et des concentrations croissantes d'anticorps anti-CD303 pendant 4 heures à 37°C. Après incubation, le surnageant est collecté. La lyse des cellules cibles induite par les anticorps anti-CD303 est mesurée de façon chromogénique en quantifiant l'enzyme intracellulaires lactate déshydrogénase (LDH) relarguée dans le surnageant par les cellules cibles lysées (Roche Diagnostics - Cytotoxicity Detection Kit LDH).

Le pourcentage de lyse est calculé selon la formule suivante :

$$\% \text{ lyse} = [(ER - SR) / (100 - SR)] - [(NC - SR) / (100 - SR)]$$

Où ER et SR représentent respectivement les relargages expérimental (ER) et spontané (SR) de LDH, et NC représente la cytotoxicité naturelle des cellules NK.Les résultats (% lyse) sont exprimés en fonction du facteur de dilution de l'anticorps. Pour chaque anticorps, la valeur d'« activité 50% » correspond au facteur de dilution de l'anticorps nécessaire pour induire 50% de la valeur plateau obtenue pour cet anticorps. Cette valeur a été calculée avec le logiciel PRISM.

Résultats

*Test de liaison à CD303*

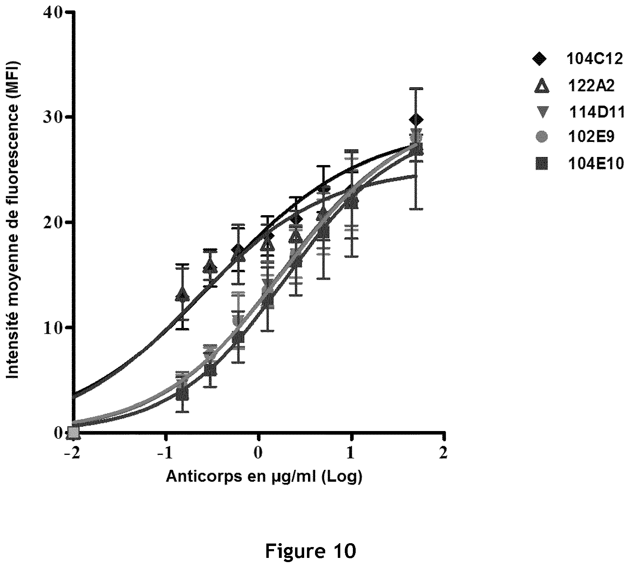

Les résultats sont présentés sur la **Figure 10**, et montrent qu'il est possible de distinguer des différences de liaison à l'antigène CD303 humain en utilisant le clone NF-3C8 ou NF-5G9 alternativement.

*Test ADCC*

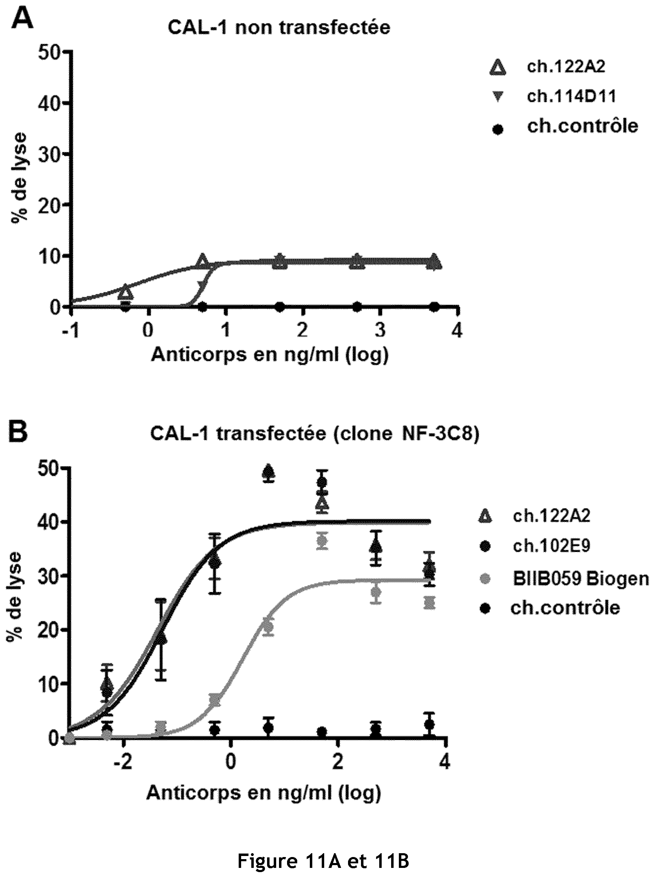

L'activité ADCC via CD16a obtenue avec les anticorps chimériques anti-CD303 122A2 et 114D1 ou avec un anticorps contrôle, en présence de la lignée CAL-1 non transfectée par un vecteur d'expression de l'antigène CD303 humain est représentée sur la **Figure 11A.** La faible activité spécifique ADCC (environ 10%) observée avec la lignée cellulaire CAL-1 est probablement due à un faible nombre de sites CD303 (entre 3000 et 6000 sites).

L'activité ADCC via CD16a obtenue avec les anticorps chimériques anti-CD303 122A2 et ch.102E9, avec l'anticorps humanisé anti-CD303 BIIB059 ou avec un anticorps contrôle, en présence de la lignée CAL-1 transfectée par un vecteur d'expression de l'antigène CD303 humain exprimant 40000 à 50000 molécules d'antigène CD303 humain à sa surface (clone NF-3C8) est représentée sur la **Figure 11B**, et dans le **Tableau 1** ci-dessous.

**Tableau 1.** Activité ADCC via CD16a obtenue avec les anticorps chimériques anti-CD303 122A2 et ch.102E9, avec l'anticorps humanisé anti-CD303 BIIB059 ou avec un anticorps contrôle. Emax : % de lyse maximale obtenue au plateau à forte concentration d'anticorps. EC50 : concentration d'anticorps permettant d'obtenir un % de lyse correspondant à 50% de la lyse maximale.

|  | Contrôle | ch.122A2 | ch.102E9 | BIIB059 |
|---|---|---|---|---|
| Emax (% lyse) | na | 39,94 | 40,19 | 29,30 |
| EC50 (ng/ml) | na | 0.04 | 0.05 | 1.68 |

Clairement, le clone NF-3C8 permet, beaucoup mieux que la lignée CAL-1 non transfectée, de mettre en évidence la réponse ADCC via CD16a induite par les différents anticorps anti-CD303.

L'activité ADCC via CD16a induite par les anticorps chimériques anti-CD303 122A2 et 114D1 est environ 40 fois plus forte que celle induite par l'anticorps humanisé anti-CD303 BIIB059.

Conclusions

Les données présentées ci-dessus montrent clairement l'intérêt des lignées selon l'invention pour caractériser et discriminer des anticorps anti-CD303 à visée thérapeutique.

**REFERENCES BIBLIOGRAPHIQUES**

Almagro et al. Frontiers in Bioscience 13, 1619-1633, January 1, 2008.
Cardarelli et al. Cancer Immunol Immunother. 2010. 59. 257-265,
Cardarelli et al. Clin Cancer Res 2009 April 28;15:3376-3383.
Dall'Acqua et al. 2002, J Immunol.; 169:5171 -80.
Dall'Acqua et al. 2006, J. Biol. Chem.;281 :23514-24. (a).
Dall'Acqua et al. J Immunol 2006; 177:1129-1138. (b).
Edelman, G.M. et al., Proc. Natl. Acad. USA, 63, 78-85 (1969).
EP1176195A1
Forthal et al, J Immunol 2010;185;6876-6882.
Herbst R. et al. J Pharmacol Exp Ther. 2010 Oct;335(1):213-22.
Hinton et al. 2004, J Biol Chem. ;279:6213-6.
Idusogie EE et al. J Immunol. 2001; 166:2571-5.
Imai-Nishiya et al, BMC Biotechnology 2007, 7:84.
Jones et al. Nature, 321 : 522-525, 1986.
JP 5011520
Kabat et al., Sequences of Proteins of Immunological Interest, 5th Ed. Public Health Service, National Institutes of Health, Bethesda, Md. (1991).
Kanda Y et al, Journal of Biotechnology 130 (2007) 300-310.
Lazar, G. A., et al. Proc Natl Acad Sci U S A. 103(11): 4005-10.
Maeda T et al., Int J Hematol. 2005 Feb ; 81(2) :148-54
Moore GL. Et al. mAbs 2:2, 181-189; March/April, 2010.
Mori K, et al. Biotechnol Bioeng. 2004 Dec 30;88(7):901-8.
Olivier S. et al. MAbs. 2010 Jul-Aug; 2(4): 405-415.
Riechmann et al. Nature, 332 : 323-327, 1988.
Shields RL, et al. J Biol Chem. 2001 Mar 2;276(9):6591-604.
Shields RL, et al. J Biol Chem. 2002 Jul 26;277(30):26733-40.
Shinkawa T, et al. J Biol Chem. 2003 Jan 31;278(5):3466-73.
Suzuki et al. Clin Cancer Res 2007 March 15;13:1875-1882.
Umana et al. Nat Biotechnol. 1999 Feb;17(2):176-80.
Verhoeyen et al. Science, 239 : 1534-1536, 1988.
WO00/42072,
WO00/42072,
WO01/77181
WO02/060919,
WO2004/029207,
WO2004/063351,
WO2004/074455,
WO2008/028686
WO2010/045193,
WO2010/106180
WO2012/041768
WO2012/080642,
WO99/51642,
Yamane-Ohnuki N. et al. Biotechnol Bioeng. 2004 Sep 5;87(5):614-22.

**Revendications**

**1.** Lignée cellulaire exprimant la chaîne gamma du récepteur FcεRI et l'antigène CD303 humain, **caractérisée en ce**

**que** ladite lignée cellulaire est transfectée de façon stable par un vecteur d'expression comprenant une molécule d'acide nucléique codant pour l'antigène CD303 humain et présente une forte expression du CD303 humain à sa surface, **caractérisée en ce que** ladite lignée cellulaire présente au moins 10000 molécules du CD303 humain par cellule et **en ce que** ladite lignée cellulaire est :

a) une lignée cellulaire de cellules dendritiques plasmacytoïdes humaines (pDC) transfectée de façon stable par un vecteur d'expression comprenant une molécule d'acide nucléique codant pour l'antigène CD303 humain ; ou

b) une lignée de lymphocyte humain, transfectée de façon stable par un vecteur d'expression comprenant une première molécule d'acide nucléique codant pour l'antigène CD303 humain et une deuxième molécule d'acide nucléique codant pour la chaine gamma du récepteur FcεRI humain, ou par deux vecteurs d'expression comprenant chacun une molécule d'acide nucléique codant respectivement pour l'antigène CD303 humain et pour la chaine gamma du récepteur FcεRI humain.

2. Lignée cellulaire selon la revendication 1, **caractérisée en ce que** ladite lignée cellulaire est une lignée transfectée de façon stable par un vecteur comprenant une molécule d'acide nucléique codant pour l'antigène CD303 humain, choisie parmi la lignée CAL-1 ou une lignée de pDC humaine obtenue à partir de cellules de néoplasme de cellules dendritiques plasmacytoïdes blastiques (BPDCN) d'un patient humain par un procédé comprenant les étapes suivantes :

• Des cellules malignes primaires de BPDCN sont obtenues à partir du sang périphérique d'un patient diagnostiqué comme souffrant de BPDCN selon la classification de l'OMS ; et
• Les cellules survivantes après culture à long terme sont clonées pour obtenir une lignée cellulaire de longue durée en culture.

3. Lignée cellulaire selon la revendication 1, **caractérisée en ce que** ladite lignée cellulaire est une lignée Jurkat, transfectée de façon stable par un vecteur d'expression comprenant une première molécule d'acide nucléique codant pour l'antigène CD303 humain et une deuxième molécule d'acide nucléique codant pour la chaine gamma du récepteur FcεRI humain, ou par deux vecteurs d'expression comprenant chacun une molécule d' acide nucléique codant respectivement pour l'antigène CD303 humain et pour la chaine gamma du récepteur FcεRI humain.

4. Lignée cellulaire selon l'une quelconque des revendications précédentes, **caractérisée en ce que** ladite lignée cellulaire présente à sa surface entre 20 000 et 60 000, préférentiellement entre 25 000 et 50 000 molécules du CD303 humain par cellule.

5. Utilisation de la lignée cellulaire selon l'une quelconque des revendications 1 à 4 pour caractériser une activité fonctionnelle d'un anticorps dirigé contre l'antigène CD303 humain, préférentiellement une activité de type ADCC, CDC, sécrétion de cytokines, apoptose, et phagocytose ou pour comparer l'affinité de liaison de plusieurs anticorps anti-CD303 à l'antigène CD303 humain ou les épitopes de l'antigène CD303 humain reconnus par plusieurs anticorps anti-CD303.

6. Utilisation de la lignée cellulaire selon l'une quelconque des revendications 1 à 4 pour caractériser les capacités fonctionnelles, notamment les capacités de type ADCC, CDC, sécrétion de cytokines, apoptose, et phagocytose, des cellules effectrices d'un sujet humain, en particulier un sujet humain souffrant de BPDCN ou d'une maladie autoimmune.

7. Procédé de test de l'activité ADCC d'un anticorps dirigé contre l'antigène CD303 humain, comprenant les étapes suivantes :

a) mise en contact de la lignée cellulaire selon les revendications 1 à 4 avec un anticorps dirigé contre l'antigène CD303 humain et des cellules effectrices ;
b) incubation pendant une durée appropriée pour permettre la lyse des cellules de la lignée cellulaire selon les revendications 1 à 4 de l'étape a); et
c) mesure du taux de lyse des cellules de la lignée cellulaire selon les revendications 1 à 4 de l'étape a).

8. Kit de caractérisation d'anticorps anti-CD303 comprenant la lignée cellulaire selon les revendications 1 à 4, et avantageusement au moins un réactif choisi parmi des cellules effectrices, des IgG polyvalentes, et des anticorps anti-CD303 de référence.

**Patentansprüche**

1. Zelllinie, die die Gamma-Kette des FcεRI-Rezeptors und das menschliche CD303-Antigen exprimiert, **dadurch gekennzeichnet, dass** die Zelllinie auf stabile Weise mit einem Expressionsvektor transfiziert ist, der ein für das menschliche CD303-Antigen kodierendes Nukleinsäuremolelkül umfasst, und eine hohe Expression des menschlichen CD303 an ihrer Oberfläche aufweist, **dadurch gekennzeichnet, dass** die Zelllinie mindestens 10000 Moleküle des menschlichen CD303 pro Zelle umfasst, und dadurch, dass die Zelllinie Folgendes ist:

   a) eine Zelllinie von menschlichen plasmazytoiden dendritischen Zellen (pDC), die auf stabile Weise mit einem Expressionsvektor transfiziert ist, der ein für das menschliche CD303-Antigen kodierendes Nukleinsäuremolekül umfasst; oder
   b) eine menschliche Lymphozytlinie, die auf stabile Weise mit einem Expressionsvektor, der ein erstes, für das menschliche CD303-Antigen kodierendes Nukleinsäuremolekül und ein zweites, für die Gamma-Kette des menschlichen FcεRI-Rezeptors kodierendes Nukleinsäuremolekül umfasst, oder mit zwei Expressionsvektoren transfiziert ist, die jeweils ein für das menschliche CD303-Antigen und für die Gamma-Kette des menschlichen FcεRI-Rezeptors kodierendes Nukleinsäuremolekül umfassen.

2. Zelllinie nach Anspruch 1, **dadurch gekennzeichnet, dass** die Zelllinie eine Linie ist, die auf stabile Weise von einem Vektor transfiziert ist, der ein für das menschliche CD303-Antigen codierendes Nukleinsäuremolekül umfasst, die unter der Linie CAL-1 oder einer menschlichen pDC-Linie gewählt ist, die ausgehend von Neoplasmazellen von blastischen plasmazytoiden dendritischen Zellen (BPDCN) eines menschlichen Patienten durch ein Verfahren erhalten wird, das die folgenden Schritte umfasst:

   • Erhalten maligner BPDCN-Primärzellen ausgehend von dem peripheren Blut eines Patienten, bei dem eine BPDCN-Erkrankung gemäß der Einstufung der WHO diagnostiziert wurde; und
   • Klonen der nach langfristiger Zellkultivierung überlebenden Zellen, um eine Langzeitkultur-Zelllinie zu erhalten.

3. Zelllinie nach Anspruch 1, **dadurch gekennzeichnet, dass** die Zelllinie eine Jurkat-Linie ist, die auf stabile Weise mit einem Expressionsvektor, der ein erstes, für das menschliche CD303-Antigen kodierendes Nukleinsäuremolekül und ein zweites, für die Gamma-Kette des menschlichen FcεRI-Rezeptors kodierendes Nukleinsäuremolekül umfasst, oder mit zwei Expressionsvektoren transfiziert ist, die jeweils ein für das menschliche CD303-Antigen kodierendes Nukleinsäuremolekül und für die Gamma-Kette des menschlichen FcεRI-Rezeptors kodierendes Nukleinsäuremolekül umfassen.

4. Zelllinie nach einem der vorhergehenden Ansprüche, **dadurch gekennzeichnet, dass** die Zelllinie an ihrer Oberfläche zwischen 20 000 und 60 000, vorzugsweise zwischen 25 000 und 50 000 Moleküle, des menschlichen CD303 pro Zelle aufweist.

5. Verwendung der Zelllinie nach einem der Ansprüche 1 bis 4 zum Charakterisieren einer funktionellen Aktivität eines gegen das menschliche CD303-Antigen gerichteten Antikörpers, vorzugsweise einer Aktivität vom Typ ADCC, CDC, Zytokinsekretion, Apoptose und Phagocytose, oder zum Vergleichen der Bindungsaffinität von mehreren Anti-CD303-Antikörpern mit dem menschlichen CD303-Antigen oder Epitopen des menschlichen CD303-Antigens, die von mehreren Anti-CD303-Antikörpern erkannt werden.

6. Verwendung der Zelllinie nach einem der Ansprüche 1 bis 4 zum Charakterisieren der funktionellen Fähigkeiten, insbesondere der Fähigkeiten vom Typ ADCC, CDC, Zytokinsekretion, Apoptose und Phagocytose, der Effektorzellen einer menschlichen Person, insbesondere einer menschlichen Person, die an BPDCN oder einer Autoimmunkrankheit erkrankt ist.

7. Verfahren zum Testen der ADCC-Aktivität eines gegen das menschliche CD303-Antigen gerichteten Antikörpers, das die folgenden Schritte umfasst:

   a) Inkontaktbringung der Zelllinie nach Anspruch 1 bis 4 mit einem gegen das menschliche CD303-Antigen gerichteten Antikörper und Effektorzellen;
   b) Inkubation während einer geeigneten Dauer, um die Lyse der Zellen der Zelllinie nach Anspruch 1 bis 4 aus dem Schritt a) zu ermöglichen; und
   c) Messung des Lyseanteils der Zellen der Zelllinie nach Anspruch 1 bis 4 von Schritt a).

**8.** Kit zur Charakterisierung von Anti-CD303-Antikörpern, das die Zelllinie nach Anspruch 1 bis 4 und vorzugsweise mindestens ein Reaktionsmittel umfasst, das zwischen Effektorzellen, polyvalenten IgG und Anti-CD303-Bezugsantikörpern gewählt ist.

**Claims**

**1.** A cell line expressing the gamma chain of Fcε receptor I (FcεRI) and human CD303 antigen, **characterized in that** said cell line is stably transfected with an expression vector comprising a nucleic acid molecule encoding human CD303 antigen and has high expression of human CD303 on its surface, **characterized in that** said cell line has at least 10,000 human CD303 molecules per cell and **in that** said cell line is:

a) a cell line of human plasmacytoid dendritic cells (pDCs) stably transfected with an expression vector comprising a nucleic acid molecule encoding human CD303 antigen; or
b) a human lymphocyte cell line, stably transfected with an expression vector comprising a first nucleic acid molecule encoding human CD303 antigen and a second nucleic acid molecule encoding the gamma chain of human FcεRI, or with two expression vectors each comprising a nucleic acid molecule encoding human CD303 antigen and the gamma chain of human FcεRI, respectively.

**2.** The cell line according to claim 1, **characterized in that** said cell line is a cell line stably transfected with a vector comprising a nucleic acid molecule encoding human CD303 antigen, selected from the CAL-1 line or a human pDC line obtained from blastic plasmacytoid dendritic cell neoplasm (BPDCN) cells from a human patient by a method comprising the following steps:

• Primary malignant BPDCN cells are obtained from peripheral blood of a patient diagnosed with BPDCN according to WHO classification; and
• Surviving cells after long-term culture are cloned to obtain a long-lasting cell line in culture.

**3.** The cell line according to claim 1, **characterized in that** said cell line is a Jurkat cell line, stably transfected with an expression vector comprising a first nucleic acid molecule encoding human CD303 antigen and a second nucleic acid molecule encoding the gamma chain of human FcεRI, or with two expression vectors each comprising a nucleic acid molecule encoding human CD303 antigen and the gamma chain of human FcεRI, respectively.

**4.** The cell line according to any one of the preceding claims, **characterized in that** said cell line has on its surface between 20,000 and 60,000, preferably between 25,000 and 50,000, human CD303 molecules per cell.

**5.** Use of the cell line according to any one of claims 1 to 4 to characterize a functional activity of an antibody against human CD303 antigen, preferably an activity of the ADCC, CDC, cytokine secretion, apoptosis or phagocytosis type, or to compare the binding affinity of several anti-CD303 antibodies to human CD303 antigen or the epitopes of human CD303 antigen recognized by several anti-CD303 antibodies.

**6.** Use of the cell line according to any one of claims 1 to 4 to characterize the functional capacities - notably capabilities of the ADCC, CDC, cytokine secretion, apoptosis and phagocytosis type - of effector cells from a human subject, in particular a human subject suffering from BPDCN or an autoimmune disease.

**7.** A method for testing the ADCC activity of an antibody against human CD303 antigen, comprising the following steps:

a) contacting the cell line according to claims 1 to 4 with an antibody against human CD303 antigen and effector cells;
b) incubating for a suitable time to allow lysis of the cells of the cell line according to claims 1 to 4 of step a); and
c) measuring the degree of lysis of the cells of the cell line according to claims 1 to 4 of step a).

**8.** A kit for characterizing anti-CD303 antibodies comprising the cell line according to claims 1 to 4, and advantageously at least one reagent selected from effector cells, polyvalent IgG, and reference anti-CD303 antibodies.

**Figure 1**

**Figure 2**

**Figure 3**

**Figure 4**

**Figure 5**

**Figure 6**

Figure 7

Figure 8

**A**

Clone 3C8 + G148

Semaine 2

Semaine 12

Événements

anti-CD303 PE

Événements

anti-CD303 PE

Clone 3C8 - G418

Semaine 2

Semaine 12

Événements

anti-CD303 PE

Événements

anti-CD303 PE

Figure 9A

**B**

Clone 5G9 + G148

Semaine 2

Événements

anti-CD303 PE

Semaine 12

Événements

anti-CD303 PE

Clone 5G9 - G418

Semaine 2

Événements

anti-CD303 PE

Semaine 12

Événements

anti-CD303 PE

Figure 9B

Figure 10

**A** CAL-1 non transfectée

△ ch.122A2
▼ ch.114D11
● ch.contrôle

**B** CAL-1 transfectée (clone NF-3C8)

△ ch.122A2
● ch.102E9
● BIIB059 Biogen
● ch.contrôle

Figure 11A et 11B

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2012080642 A
- WO 2014093396 A1
- JP 5011520 B
- WO 0177181 A
- EP 1176195 A1
- WO 2012041768 A
- WO 2008028686 A
- WO 0042072 A
- WO 2004029207 A
- WO 2004063351 A
- WO 2004074455 A
- WO 02060919 A
- WO 2010045193 A
- WO 2010106180 A
- WO 9951642 A

**Littérature non-brevet citée dans la description**

- **PELLERIN et al.** *EMBO Mol Med,* 2015, vol. 7, 464-476
- **MAEDA T et al.** *Int J Hematol.,* Février 2005, vol. 81 (2), 148-54
- **T et al.** *Int J Hematol.,* Février 2005, vol. 81 (2), 148-54
- **ALMAGRO et al.** *Frontiers in Bioscience,* 01 Janvier 2008, vol. 13, 1619-1633
- **CARDARELLI et al.** *Cancer Immunol Immunother.,* 2010, vol. 59, 257-265
- **CARDARELLI et al.** *Clin Cancer Res,* 28 Avril 2009, vol. 15, 3376-3383
- **DALL'ACQUA et al.** *J Immunol.,* 2002, vol. 169, 5171-80
- **DALL'ACQUA et al.** *J. Biol. Chem.,* 2006, vol. 281, 23514-24
- **DALL'ACQUA et al.** *J Immunol,* 2006, vol. 177, 1129-1138
- **EDELMAN, G.M. et al.** *Proc. Natl. Acad. USA,* 1969, vol. 63, 78-85
- **FORTHAL et al.** *J Immunol,* 2010, vol. 185, 6876-6882
- **HERBST R. et al.** *J Pharmacol Exp Ther.,* Octobre 2010, vol. 335 (1), 213-22
- **HINTON et al.** *J Biol Chem.,* 2004, vol. 279, 6213-6
- **IDUSOGIE EE et al.** *J Immunol.,* 2001, vol. 166, 2571-5
- **IMAI-NISHIYA et al.** *BMC Biotechnology,* 2007, vol. 7, 84
- **JONES et al.** *Nature,* 1986, vol. 321, 522-525
- **KABAT et al.** Sequences of Proteins of Immunological Interest. Public Health Service, National Institutes of Health, 1991
- **KANDA Y et al.** *Journal of Biotechnology,* 2007, vol. 130, 300-310
- **LAZAR, G. A. et al.** *Proc Natl Acad Sci U S A.,* vol. 103 (11), 4005-10
- **MOORE GL. et al.** *mAbs,* Mars 2010, vol. 2 (2), 181-189
- **MORI K et al.** *Biotechnol Bioeng.,* 30 Décembre 2004, vol. 88 (7), 901-8
- **OLIVIER S. et al.** *MAbs,* Juillet 2010, vol. 2 (4), 405-415
- **RIECHMANN et al.** *Nature,* 1988, vol. 332, 323-327
- **SHIELDS RL et al.** *J Biol Chem.,* 02 Mars 2001, vol. 276 (9), 6591-604
- **SHIELDS RL et al.** *J Biol Chem.,* 26 Juillet 2002, vol. 277 (30), 26733-40
- **SHINKAWA T et al.** *J Biol Chem.,* 31 Janvier 2003, vol. 278 (5), 3466-73
- **SUZUKI et al.** *Clin Cancer Res,* 15 Mars 2007, vol. 13, 1875-1882
- **UMANA et al.** *Nat Biotechnol.,* Février 1999, vol. 17 (2), 176-80
- **VERHOEYEN et al.** *Science,* 1988, vol. 239, 1534-1536
- **YAMANE-OHNUKI N. et al.** *Biotechnol Bioeng.,* 05 Septembre 2004, vol. 87 (5), 614-22